# EUROPEAN PATENT APPLICATION

(11) **EP 0 597 401 A2**
(43) Date of publication of application: **18.05.1994**
(21) Application number: 93117994.9
(22) Date of filing: 05.11.1993
(51) Int. Cl.: C07D 499/88, C07D 205/09, C07D 401/12, C07F 9/28, C07D 417/12, C07F 7/10, C07D 487/04

(54) **Production of penem**

(30) Priority: 11.11.1992 JP 301384/92; 25.12.1992 JP 347204/92; 16.04.1993 JP 90022/93
(71) Applicant: Takeda Chemical Industries, Ltd., Osaka-shi, Osaka 541 (JP)
(72) Inventor: Maeda, Yoshiharu, Osaka 584 (JP); Akita, Masahiro, Mishima-gun, Osaka 618 (JP); Kurizono, Kunimitsu, Minoo, Osaka 562 (JP); Nakai, Shinobu, Takatsuki, Osaka 569 (JP); Mizuno, Masahiro, Takatsuki, Osaka 569 (JP); Inagaki, Atsushi, Ikeda, Osaka 563 (JP)
(74) Representative: Lederer, Franz, Dr.

(57) **Abstract**

A process for producing a compound of the formula: 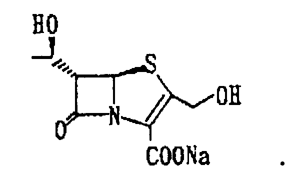
wherein R¹ represents a hydrogen atom or a hydroxy-protecting group, R² represents an organic group and R^{3a} represents a hydrogen atom or an acetoxymethyl group or a salt thereof, having a broad antibacterial spectrum and being of value as an antibacterial agent, and processes for producing intermediates used in the preparation of the compound (I) or a salt thereof, with remarkable industrial advantages.

## Description

The present invention relates to a remarkably industrially advantageous process for producing a compound of the formula: 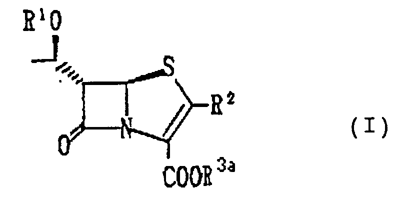
wherein R¹ represents a hydrogen atom or a hydroxy-protecting group, R² represents an organic group and R^{3a} represents a hydrogen atom or an acetoxymethyl group, or a salt thereof, which has a broad antibacterial spectrum and is of value as an antibacterial agent as stated in U.S. Patent 4,634,556, U.S. Patent 4,826,832 and EP-A-O 499 274.

This invention relates also to remarkably industrially advantageous processes for producing intermediates including novel compounds used in the preparation of the compound (I) or a salt thereof.

### A. Production of the Compound (I) or a Salt Thereof

The present invention in a first aspect relates to a commercially useful process for producing penem compounds (I) and their salts which have antimicrobial activity and are of value as medicines.

Penem antibiotics each having a broad antibacterial spectrum have for some time been attracting attention as antibacterial agents and a variety of processes for synthesizing them have been developed.

In these processes for synthesizing penem compounds, an allyl group is generally used for the protection of the 3-carboxy group but because of the instability of penem compounds to acids and alkalies, removal of the introduced allyl group has to be carried out under neutral conditions. For this reason, the deprotection reaction is generally carried out using tetrakis(triphenylphosphine)palladium as the catalyst. For example, U.S. Patent 4,997,829 describes a process comprising permitting either a triarylphosphine and palladium tetrakis(triallylphosphine) or tributyltin hydride and palladium tetrakistriallylphosphine to act upon a compound of the formula: 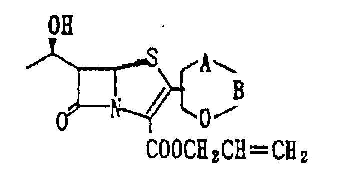
wherein -A- represents an oxygen atom or a methylene group; -B- represents a methylene, ethylene or carbonyl group, to provide a compound of the formula: 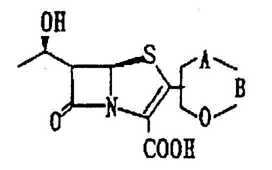
wherein all the symbols have the meanings defied above, or a salt thereof.

U.S. Patent 4,782,146 describes, in Example 6 thereof, a process comprising permitting triphenylphosphine, sodium 2-ethylhexanoate and tetrakis(triphenylphosphine)palladium to act on a compound of the formula: 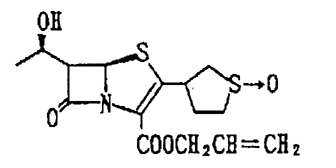
to provide a compound of the formula:

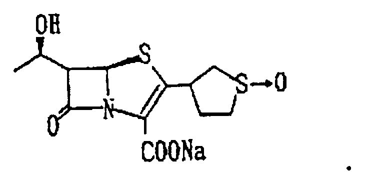

EPC-A-462521 describes a process comprising permitting triphenylphosphine, potassium 2-ethylhexanoate and tetrakis(triphenylphosphine)palladium to act on a compound of the formula: 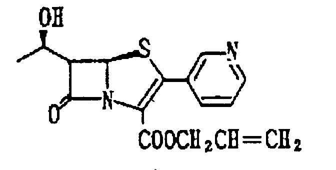
to provide a compound of the formula:

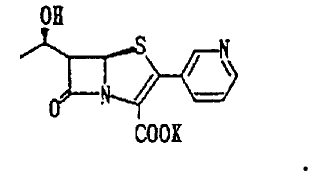

Tetrahedron Letters 33, 3733-3736 (1992) describes a process comprising subjecting a compound of the formula: 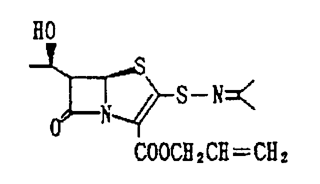
to a catalytic conversion reaction in the presence of sodium 2-ethylhexanoate and tetrakis(triphenylphosphine)palladium to provide a compound of the formula:

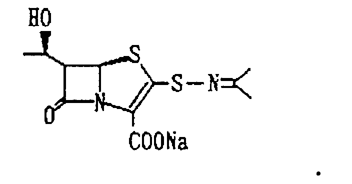

Journal of Organic Chemistry 47, 587-590 (1982) describes a process comprising permitting triphenylphosphine, potassium 2-ethylhexanoate and tetrakis(triphenylphosphine)palladium to act upon a compound of the formula: 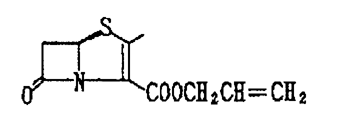
to provide a compound of the formula:

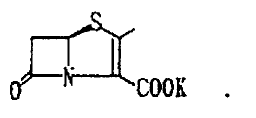

Journal of Medicinal Chemistry 35, 1828-1839 (1992) describes a process in which a compound of the formula: 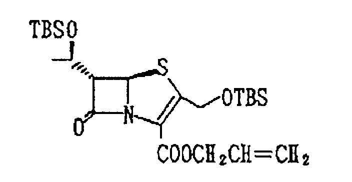
wherein TBS stands for tert-butyldimethylsilyl group, is treated with triphenylphosphine, sodium 2-ethylhexanoate and tetrakis(triphenylphosphine)palladium to provide a compound of the formula:

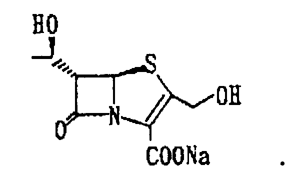

Thus, in the field of synthesis of penem compounds, it is common practice to employ tetrakis(triphenylphosphine)palladium as a catalyst for removal of the allyl group protecting the 3-carboxyl function.

However, tetrakis(triphenylphosphine)palladium which, as aforesaid, is used as the catalyst in the above-mentioned prior art processes for providing penem compounds is so unstable that it is readily deactivated when allowed to stand in the air, thus calling for great agility in handling. Moreover, this compound undergoes deactivation with time even when maintained in a nitrogen atmosphere as is usually done, thus having a serious disadvantage as the catalyst for the mass production of penem compounds.

Meanwhile, there is not known a technology for removing the allyl group protecting the 3-carboxy function of a penem compound without enlisting the help of tetrakis(triphenylphosphine)palladium.

Much awaited, therefore, is the development of an agent that could be used as an effective catalyst for the elimination of the 3-carboxy-protecting allyl group of penems which are labile to acids and alkalis, and might be easily handled and little susceptible to the aging of catalytic activity.

After an assiduous research endeavor to solve the above problems it was discovered that when a compound of the formula: 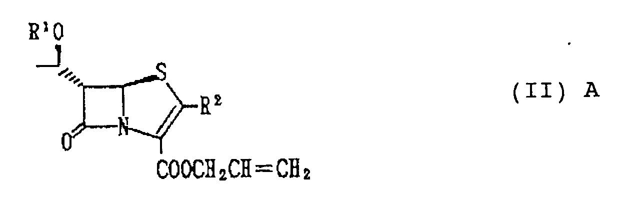
wherein R¹ represents a hydrogen atom or a hydroxy-protecting group and R² represents an organic group, is reacted with an allyl acceptor in the presence of a divalent palladium salt and a reducing agent for the salt, a compound of formula: 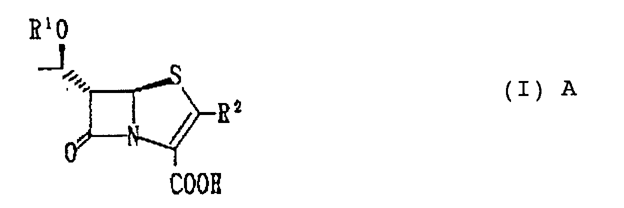
wherein all the symbols have the meanings defined above or a salt thereof is obtained in high purity and good yield with a remarkable industrial advantage. The present invention has been developed on the basis of the above finding.

The aspect of the present invention includes:-
(1) a process for producing a compound (I) A or a salt thereof, characterized in that a compound (II) A is reacted with an allyl acceptor in the presence of a divalent palladium salt and a reducing agent for the salt;
(2) a process according to (1) wherein said divalent palladium salt is palladium acetate or palladium chloride;
(3) a process according to (1) wherein the reaction is carried out in a solvent comprising tetrahydrofuran and methylene chloride;
(4) a process according to (1) wherein said allyl acceptor is an alkali metal 2-alkylhexanoate;
(5) a process according to (1) wherein said allyl acceptor is potassium 2-ethylhexanoate;
(6) a process according to (1) wherein R¹ represents a hydrogen atom;
(7) a process according to (1) wherein R² represents a pyridyl group;
Referring to the above formulas, R¹ represents H or a hydroxy-protecting group. As the hydroxy-protecting group, those protective groups which are generally used in peptide chemistry, β-lactam chemistry, etc. can be selectively employed. Thus, among such groups may be used C₁₋₇ acyl groups which may be substituted by one to three groups selected from halogen (e.g. chloro, bromo, fluoro, etc.), nitro and the like, such as formyl, acetyl, chloroacetyl, dichloroacetyl, trichloroacetyl, trifluoroacetyl, propionyl, allyloxyglyoxyloyl, 4-nitrobenzoyl, 2-nitrobenzoyl, etc.; C₇₋₁₉ aralkyl groups which may be substituted by one to three selected from C₁₋₄ alkoxy (e.g. methoxy, ethoxy, etc.), nitro and the like, such as benzyl, 4-methoxybenzyl, 2-nitrobenzyl, 4-nitrobenzyl, diphenylmethyl, triphenylmethyl, etc.; C₁₋₁₆ alkyloxy-carbonyl groups which may be substituted by one to three groups selected from C₁₋₄ alkoxy (e.g. methoxy, ethoxy, etc.), halogen (e.g. chloro, bromo, etc.), tri-C₁₋₄ alkylsilyl (e.g. trimethylsilyl etc.), etc., such as methoxycarbonyl, ethoxycarbonyl, t-butoxycarbonyl, 2,2,2-trichloroethoxycarbonyl, 2-trimethylsilylethoxycarbonyl, etc.; C₇₋₁₉ aralkyloxycarbonyl groups which may be substituted by one to three substituents selected from C₁₋₄ alkoxy (e.g. methoxy, ethoxy, etc.), nitro, etc. such as benzyloxycarbonyl, 4-methoxybenzyloxycarbonyl, 3,4-dimethoxybenzyloxycarbonyl, 2-nitrobenzyloxycarbonyl, 4-nitrobenzyloxycarbonyl, etc.; C₂₋₆ alkenyloxycarbonyl groups such as vinyloxycarbonyl, allyloxycarbonyl, etc.; substituted methyl groups as substituted by 1 to 3 of C₁₋₄ alkoxy (e.g. methoxy, ethoxy, etc.) groups which, in turn, may be substituted by one to three halogens (e.g. chloro, bromo, fluoro, etc.), such as methoxymethyl, t-butoxymethyl, 2-methoxyethoxymethyl, 2,2,2-trichloroethoxymethyl, etc.; substituted ethyl groups as substituted by one to three substituents selected from C₁₋₄ alkoxy (e.g. methoxy, ethoxy, etc.), halogen (e.g. chloro, bromo, fluoro, etc.), etc., such as 1-ethoxyethyl, 1-methyl-1-methoxyethyl, 2,2,2-trichloroethyl, etc.; tri-C₁₋₄ alkylsilyl groups such as trimethylsilyl, triethylsilyl, dimethylisopropylsilyl, t-butyldimethylsilyl, triisopropylsilyl, etc.; and diphenyl-C₁₋₄ alkylsilyl groups such as diphenylmethylsilyl, diphenylethylsilyl and so on. Preferred, among them, are C₇₋₁₉ aralkyloxycarbonyl groups (e.g. benzyloxycarbonyl, etc.), C₂₋₆ alkenyloxycarbonyl group (e.g. vinyloxycarbonyl, etc.), tri-C₁₋₄ alkylsilyl groups (e.g. trimethylsilyl, etc.), etc. Still more preferred are tri-C₁₋₄ alkylsilyl groups, etc., particularly tert-butyldimethylsilyl, etc.

Preferably, R¹ is a hydrogen atom or a tri-C₁₋₄ alkylsilyl group such as tert-butyldimethylsilyl. More preferably, R¹ is H or tert-butyldimethylsilyl.

Referring, further, to the above formulas, R² represents an organic group. The organic group includes a group of the formula -W-R²' wherein W represents a bond, N, O or S and R²' represents a hydrocarbon group which may be substituted or a heterocyclic group which may be substituted.

The hydrocarbon moiety of this optionally substituted hydrocarbon group R 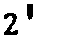 includes, among others, (1) saturated or unsaturated C₁₋₆ aliphatic hydrocarbon groups, (2) C₃₋₈ alicyclic hydrocarbon groups and (3) C₆₋₁₄ aromatic hydrocarbon groups. Preferred are C₁₋₆ alkyl groups, C₂₋₆ alkenyl groups, C₂₋₆ alkynyl groups, C₃₋₈ cycloalkyl groups, C₆₋₁₄ aryl groups, etc. Still more preferred are C₁₋₆ alkyl groups, C₆₋₁₄ aryl groups, etc.

The C₁₋₆ alkyl groups mentioned above include methyl, ethyl, propyl, isopropyl, butyl, isobutyl, sec-butyl, tert-butyl, etc. Preferably, C₁₋₄ alkyl groups such as methyl, ethyl, propyl, isopropyl, etc. are employed.

The C₂₋₆ alkenyl groups include ethenyl, propenyl, isopropenyl, butenyl, isobutenyl, sec-butenyl, etc. Preferably, C₂₋₄ alkenyl groups such as ethenyl, propenyl, isopropenyl, etc. are employed.

The C₂₋₆ alkynyl groups include ethynyl, propynyl, isopropynyl, butynyl, isobutynyl, sec-butynyl, etc. Preferred are C₂₋₄ alkynyl groups such as ethynyl, propynyl, isopropynyl, etc.

The C₃₋₈ cycloalkyl groups include cyclopropyl, cyclobutyl, cyclopentyl, cyclohexyl, etc. Preferred are C₃₋₆ cycloalkyl groups such as cyclopropyl, cyclobutyl, etc.

The C₆₋₁₄ aryl groups include phenyl, naphthyl, anthryl, phenanthryl, etc. Preferred are C₆₋₁₀ aryl groups such as phenyl and naphthyl.

The heterocyclic moiety of the optionally substituted heterocyclic group R²' includes, among others, 5- or 6-membered heterocyclic groups containing 1 to 3 nitrogen, sulfur and/or oxygen atoms in addition to carbon atoms. Preferred are 5-membered heterocyclic groups containing one nitrogen, sulfur or oxygen atom, such as pyrrole, pyrroline, thienyl, furyl, etc.; nitrogen-containing 6-membered heterocyclic groups such as 6-membered heterocyclic groups containing 1 or 2 nitrogen atoms in addition to carbon atoms, e.g. pyridyl, pyrimidyl, pyridazinyl, etc. and nitrogen-containing 5-membered heterocyclic groups such as 5-membered heterocyclic groups containing 2 nitrogen atoms in addition to carbon atoms, e.g. pyrazolyl, imidazolyl, etc., 5-membered heterocyclic groups containing 1 nitrogen atom and 1 sulfur atom in addition to carbon atoms, e.g. thiazolyl etc., 5-membered heterocyclic groups containing 2 nitrogen atoms and 1 sulfur atom in addition to carbon atoms, e.g. 1,2,3-thiadiazolyl, 1,2,4-thiadiazolyl, 1,3,4-thiadiazolyl, 1,2,5-thiadiazolyl, etc., 5-membered heterocyclic groups containing 2 nitrogen atoms and 1 oxygen atom in addition to carbon atoms, e.g. 1,2,3-oxadiazolyl, 1,2,4-oxadiazolyl, 1,3,4-oxadiazolyl, 1,2,5-oxadiazolyl, etc., 5-membered heterocyclic groups containing 3 nitrogen atoms in addition to carbon atoms, e.g. 1,2,5-triazolyl, 1,2,4-triazolyl, etc., and 5-membered groups containing 4 nitrogen atoms in addition to a carbon atom, e.g. 1H-tetrazolyl, 2H-tetrazolyl, etc. More preferred are 5-membered heterocyclic groups containing one nitrogen, sulfur or oxygen atom in addition to carbon atoms and 6-membered heterocyclic groups containing 1 or 2 nitrogen atoms in addition to carbon atoms. Particularly preferred are thienyl, furyl and pyridyl.

The substituents on the above optionally substituted hydrocarbon group and optionally substituted heterocyclic group, represented by R²', include, among others, (1) hydroxy, (2) oxo, (3) mercapto, (4) halogen, (5) carboxy, (6) carbamoyl, (7) carbamoyloxy, (8) alkoxy, (9) alkoxycarbonyl, (10) alkylthio, (11) alkanoyl, (12) alkanoyloxy, (13) amino optionally substituted by one or two of alkyl and alkanoyl, where the substituents may be the same or different, (14) cycloalkyl, (15) aryl and (16) heterocyclic groups, and where the hydrocarbon group is an alicyclic hydrocarbon group or an aromatic hydrocarbon group, (17) alkyl groups optionally substituted by 1 or 2 members, which may be the same or different, of hydroxy, halogen, amino and carboxy. The number of the substituent is one to five, preferably one or two in the possible position(s).

The halogen mentioned above includes chloro, fluoro, bromo and iodo and may preferably be chloro or fluoro.

The alkoxy group includes C₁₋₆ alkoxy groups such as methoxy, ethoxy, propoxy, isopropoxy, butoxy, isobutoxy, sec-butoxy, tert-butoxy, etc. Preferred are C₁₋₄ alkoxy groups such as methoxy, ethoxy, propoxy, isopropoxy, etc.

The alkoxy moiety of the alkoxycarbonyl group includes the alkoxy groups mentioned above.

The alkyl moiety of the alkylthio group includes the alkyl groups mentioned above for the hydrocarbon group of R²'.

The alkanoyl group includes C₁₋₆ alkanoyl groups such as acetoxy, pivaloyloxy, etc. Preferred are C₁₋₄ alkanoyl groups such as acetoxy, etc.

The alkanoyl moiety of the alkanoyloxy group includes the alkanoyl groups mentioned above.

The alkyl or alkanoyl moiety of the optionally substituted amino group as substituted by 1 or 2 members, which may be the same or different, of alkyl and alkanoyl groups includes the above-mentioned groups. Examples of the optionally substituted amino group include C₁₋₆ alkylamino, di-C₁₋₆ alkylamino and C₁₋₆ alkanoylamino groups such as methylamino, ethylamino, dimethylamino, diethylamino, methylethylamino, acetoxyamino, etc. Preferred are C₁₋₄ alkylamino, di-C₁₋ ₄ alkylamino and C₁₋₄ alkanoylamino groups such as methylamino, ethylamino, dimethylamino, diethylamino, acetoxyamino, etc.

The cycloalkyl, aryl and heterocyclic groups include the same groups as mentioned previously for R² representing a hydrocarbon or heterocyclic group.

The halogen of the optionally substituted alkyl group as substituted by 1 or 2 members, which may be the same or different species, of hydroxy, halogen, amino and carboxy includes the same halogen species as mentioned hereinbefore. Similarly, the alkyl moiety of said optionally substituted alkyl group includes the groups mentioned above for said hydrocarbon group of R²'. Examples of the optionally substituted alkyl group include methyl, ethyl, hydroxymethyl, hydroxyethyl, hydroxypropyl, chloromethyl, chloroethyl, fluoromethyl, fluoroethyl, aminomethyl, aminoethyl, carboxymethyl, carboxyethyl, etc. Preferred are methyl, ethyl, hydroxymethyl, fluoromethyl, aminomethyl, carboxymethyl, etc.

The preferred substituent on the hydrocarbon group which may be substituted or on the heterocyclic group which may be substituted includes, among others, (a) hydroxy, (b) oxo, (c) halogen, (d) carboxy, (e) carbamoyl, (f) carbamoyloxy, (g) C₁₋₄ alkoxy, (h) amino, (i) C₁₋₆ alkyl which may be substituted by 1 or 2 identical or different members of hydroxy, halogen, amino and/or carboxy, etc. Still more preferred are (a) hydroxy, (b) oxo, (e) carbamoyl, (f) carbamoyloxy, (g) C₁₋₄ alkoxy, (h) amino, (i) C₁₋₄ alkyl, etc.

The preferred species of R² includes, among others, (i) C₁₋₆ alkyl groups which may be substituted by a carbamoyl, carbamoyloxy or C₁₋₄ alkoxy, (ii) C₆₋₁₀ aryl groups which may be substituted by a carbamoyl, carbamoyloxy or C₁₋₄ alkoxy; (iii) nitrogen-, sulfur- or oxygen-containing 5-membered heterocyclic groups which may be bound through N, O or S and be substituted by an oxo, carbamoyl, amino or C₁₋₄ alkyl groups; (iv) 6-membered heterocyclic groups containing 1 or 2 nitrogen atoms, etc. More preferred are C₁₋₆ alkyl groups which may be substituted by a carbamoyloxy or C₁₋₄ alkoxy; C₆₋ ₁₀ aryl groups which may be substituted by a carbamoyl; nitrogen-, sulfur- or oxygen-containing 5-membered heterocyclic groups which may be bound through S and be substituted by an oxo; 6-membered heterocyclic groups containing 1 or 2 nitrogen atoms, etc. Particularly preferred are C₁₋₄ alkoxy-C₁₋₄ alkyl, carbamoyloxy-C₁₋₄ alkyl, carbamoyl-C₆₋₁₀ aryl, thienylthio, S-oxidothienylthio, furyl, pyridyl, etc.

The allyl acceptor for use in the process of this aspect of the invention includes, among others, alkali metal salts of 2-alkylhexanoic acids, alkali metal enolates of 1,3-diketones, and tertiary amines.

Among said alkali metal salts of 2-alkylhexanoic acids may be used alkali metal salts of 2-C₁₋₄ alkylhexanoic acids, such as lithium 2-methylhexanoate, sodium 2-methylhexanoate, potassium 2-methylhexanoate, lithium 2-ethylhexanoate,sodium 2-ethylhexanoate, potassium 2-ethylhexanoate,sodium 2-propylhexanoate, potassium 2-propylhexanoate, etc. Preferred are alkali metal salts of 2-ethylhexanoic acid, such as sodium 2-ethylhexanoate, potassium 2-ethylhexanoate, etc. Still more preferred is potassium 2-ethylhexanoate or the like. Included among such alkali metal salts of 2-alkylhexanoic acids are the products of reaction of 2-alkylhexanoic acids with alkali metal hydroxides in a solvent.

The alkali metal enolates of 1,3-diketones include, among others, compounds of the formula: 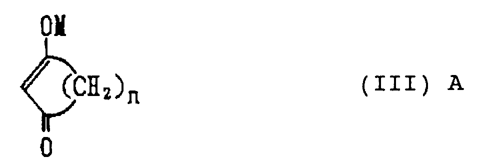
wherein M represents an alkali metal and n represents a whole number of 2 through 4; the ring hydrogen may be substituted by lower alkyl, lower alkyloxycarbonyl, halogen or aryl. Preferred are compounds in which M represents sodium or potassium. The lower alkyl group or the lower alkyl moiety of the lower alkyloxycarbonyl group includes C₁₋₄ alkyl groups such as methyl, ethyl, propyl, isopropyl, etc. The halogen includes chloro, fluoro, bromo and iodo, and may preferably be chloro or fluoro. The aryl group includes C₁₋₆ aryl groups such as phenyl, naphthyl, etc. As such compounds (III) A, there can be used sodium 1,3-cyclohexanedione enolate, sodium dimedone enolate, sodium 5,5-dimethyl-4-methoxycarbonyl-1,3-cyclohexanediolate, potassium 1,3-cyclohexanediol enolate, potassium dimedone enolate, and potassium 5,5-dimethyl-4-methoxycarbonyl-1,3-cyclohexanediolate and so on. Preferred is potassium dimedone enolate, etc.

These alkali metal enolates of 1,3-diketone compounds include an aqueous solution containing the corresponding 1,3-diketone and alkali metal.

The tertiary amine includes, among others, N-methylmorpholine, N-methylpyrrolidine, N-methylpiperazine, tributylamine, triethylamine, etc. and is preferably N-methylmorpholine or the like.

The preferred allyl acceptor includes, among others, alkali metal 2-ethylhexanoates such as potassium 2-ethylhexanoate etc., potassium dimedone enolate and N-methylmorpholine and is more preferably potassium 2-ethylhexanoate or the like.

The divalent palladium salt for use in the process of this aspect of the invention includes palladium halides such as palladium fluoride, palladium chloride, palladium bromide and palladium iodide, and organic or inorganic acid salts of palladium such as palladium acetate, palladium nitrate, palladium sulfate, etc. and is preferably palladium chloride, palladium acetate or the like.

The reducing agent for said divalent palladium salt, which is used in the process of this aspect of the invention, includes tri-C₁₋₄ alkyl phosphites such as trimethyl phosphite, triethyl phosphite, tripropyl phosphite, tributyl phosphite, etc. and tri-C₆₋₁₀ arylphosphines such as triphenylphosphine, etc. and is preferably a tri-C₁₋₄ alkyl phosphite such as triethyl phosphite, etc. More preferred is triethyl phosphite or the like.

In the process of this aspect of the invention, the ratio of allyl acceptor to compound (II) A is about 1 to 4 moles, preferably about 1 to 2 moles, of allyl acceptor per mole of compound (II) A. The ratio of divalent palladium salt to compound (II) A is about 0.0001 to 0.1 mole, preferably about 0.001 to 0.05 mole, of the salt to each mole of compound (II) A. The ratio of reducing agent to divalent palladium salt is about 2 to 20 moles, preferably about 3 to 7 moles, of reducing agent per mole of divalent palladium salt.

In the process of this aspect of the invention, the "divalent palladium salt and reducing agent therefor" may be a reaction mixture comprising a divalent palladium salt and a reducing agent and includes the case in which the divalent palladium salt and reducing agent react with each other in the course of the reaction of this aspect of the present invention.

In the process of this aspect of the invention, the reaction temperature is about -20°C to 50°C and preferably about 0°C to 40°C and the reaction time is about 5 minutes to 8 hours and preferably about 5 minutes to 5 hours.

The reaction is generally conducted in a solvent. The solvent is selected from among those solvents which are capable of dissolving said compound (II) A, allyl acceptor, divalent palladium salt and reducing agent. As the solvent, an ether such as tetrahydrofuran (briefly, THF), a halogenated hydrocarbon such as methylene chloride or a mixture thereof is employed. The preferred solvent includes a mixture of THF and methylene chloride (1:0.1 ^{∼} 10, preferably 1:0.5 ^{∼} 2, v/v). The ratio (v/w) of the solvent to compound (II) A is about 3 to 30 v/w and preferably about 5 to 20 v/w.

The compound (I) A produced by the process of this aspect of the invention is usually in the form of a salt when isolated as a solid but can be isolated in the free acid form by the conventional procedure. Where compound (I) A forms a salt, the species of salt may be determined according to the species of allyl acceptor. For example, where the allyl acceptor is an alkali metal 2-alkylhexanoate or an alkali metal 1,3-diketone enolate, the compound forms a salt with the alkali metal of the allyl acceptor used. Where the allyl acceptor is a tertiary amine, compound (I) A may form a salt with the quaternary amine produced as the tertiary amine accepts the allyl group.

The resulting compound (I) A can be separated and purified by the conventional isolation procedures such as concentration, extraction, washing, chromatography, crystallization and recrystallization but can be used directly, i.e. as it is without isolation, as the starting material for producing a compound (I) wherein R^{3a} represents an acetoxymethyl group.

The production of the compound (I) wherein R^{3a} represents an acetoxymethyl group can be conducted by reacting the compound (I) A or a salt thereof with a halogenomethyl acetate in a manner like that of the reaction of the compound (X) A with bromomethyl acetate as mentioned hereinafter. And, if necessary, before or after the production reaction of the compound (I) wherein R^{3a} represents an acetoxymethyl group, the protecting group can be removed by the conventional manner.

The resulting compound (I) wherein R^{3a} represents an acetoxymethyl group can be separated and purified by the conventional isolation procedures as mentioned above.

The starting compound (II) A can be synthesized by the processes described or suggested in the next aspect of this invention.

By way of illustration, the compound of formula: 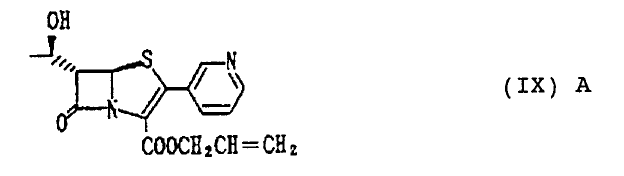
, which is subsumed in the category of starting compound (II) A for this aspect of the invention, can be synthesized from (3R,4R)-4-acetoxy-3-[(R)-1-(tert-butyldimethylsilyloxy)ethyl]-2-azetidinone of the formula: 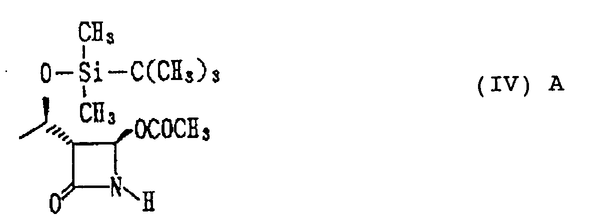
, which is an intermediate of penem antibiotics, in accordance with the following reaction schema. 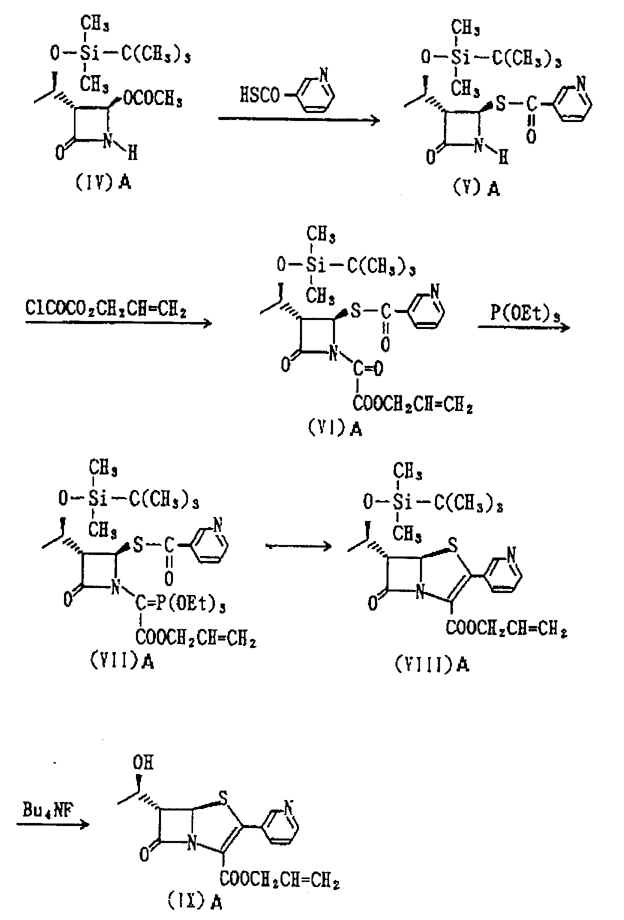
Thus, compound (IV) A is reacted with thionicotinic acid to substitute an acetoxy group with a nicotinoylthio group, giving rise to compound (V) A. This reaction is carried out in a binary solvent system comprising an inert organic solvent (e.g. dichloromethane, ethyl acetate, toluene, etc.) and water, preferably dichloromethane-water. Thionicotinic acid is used in a proportion of about 1 to 3 moles, preferably about 1 to 1.5 moles, per mole of compound (IV) A. The reaction temperature is about 0°C to 40°C and preferably about 10°C to 30°C. The reaction time is about 0.5 to 30 hours and preferably about 1 to 24 hours.

Compound (V) A is converted to compound (VI) A by introducing an allyloxyglyoxyloyl group through reaction with allyloxyoxalyl chloride in the presence of a tri-lower-alkylamine, such as triethylamine, N,N-diisopropylethylamine, etc., preferably triethylamine or N,N-diisopropylethylamine. The allyloxyoxalyl chloride is used in a proportion of about 1 to 2 moles, preferably about 1 to 1.5 moles, per mole of compound (V) A. This reaction is carried out in an inert organic solvent (e.g. dichloromethane, ethyl acetate, toluene, etc.), preferably in dichloromethane. The reaction temperature is about -40°C to 30°C and preferably about -20°C to 20°C. The reaction time is not more than 2 hours and preferably about 3 to 90 minutes.

The resulting compound (VI) A is reacted with triethyl phosphite to give compound (VII) A. This reaction may be carried out in the absence of a solvent or in an inert organic solvent (e.g. dichloromethane, xylene, toluene, dioxane, etc.). Triethyl phosphite is used in a proportion of generally about 2 to 50 moles and preferably about 5 to 30 moles per mole of compound (VI) A. The reaction temperature is about 20 to 80°C and preferably about 40 to 60°C. The reaction time is about 0.5 to 10 hours and preferably about 1 to 4 hours.

Compound (VII) A is converted to compound (VIII) A by heating in 2-methyl-1-propyl alcohol. The concentration of compound (VII) A in the alcohol is about 1 to 50% (w/w) and preferably about 5 to 30% (w/w). The heating temperature is about 30 to 100°C and preferably about 50 to 90°C. The reaction time is about 30 minutes to 5 hours and preferably about 1 to 3 hours.

Then, compound (VIII) A is reacted with tetrabutylammonium fluoride in the presence of acetic acid to eliminate the tert-butyldimethylsilyl group protecting the hydroxyl function and thereby provide compound (IX) A. This reaction is conducted in an inert organic solvent such as THF, dichloromethane, acetonitrile, etc., preferably in THF. Tetrabutylammonium fluoride is used in a proportion of about 1 to 20 moles, preferably about 2 to 10 moles, per mole of compound (VIII) A. The reaction temperature is about 0°C to 50°C and preferably about 15°C to 30°C. The reaction time is about 30 minutes to 40 hours and preferably about 5 to 30 hours.

Compound (IX) A, thus obtained, can be subjected to a reaction for elimination of the allyl group and conversion to a penem ester by the method of this aspect of the present invention as shown schematically below. 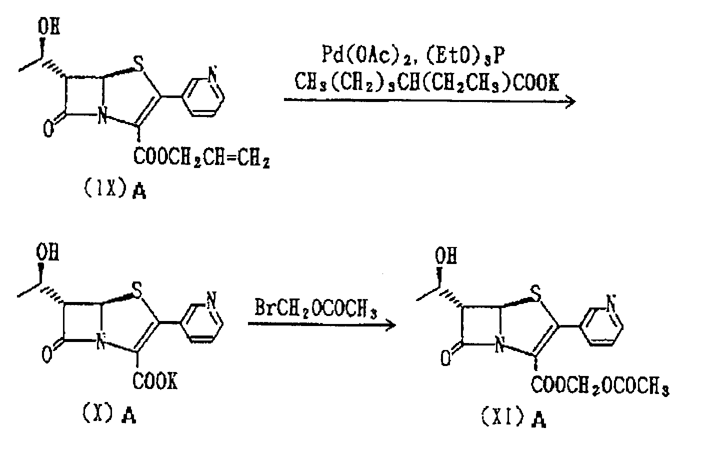
Thus, in accordance with the process of this aspect of the invention, compound (IX) A is reacted with potassium 2-ethylhexanoate in the presence of palladium acetate and triethyl phosphite to remove the carboxy-protecting allyl group and thereby provide a penem potassium salt (X) A. Potassium 2-ethylhexanoate is used in a proportion of about 1 to 4 moles, preferably about 1 to 2 moles, per mole of compound (IX) A. Palladium acetate is used in a catalytic amount, namely about 0.0001 to 0.1 mole and preferably about 0.001 to 0.05 moles, per mole of compound (IX) A. Triethyl phosphite is used in a proportion of about 2 to 20 moles, preferably about 3 to 7 moles, per mole of palladium acetate. The reaction is carried out in a solvent. The solvent can be virtually any type of solvent that does not interfere with the reaction. For example, this reaction can be carried out in an inert organic solvent such as THF, methylene chloride or a mixture thereof, preferably a mixture of THF and methylene chloride. The reaction temperature is about -20°C to 50°C and preferably about 0°C to 40°C. The reaction time is about 5 minutes to 8 hours and preferably about 15 minutes to 5 hours. The resulting compound (X) A can be separated and purified by the conventional isolation procedures.

The compound (X) A thus obtained can be reacted with bromomethyl acetate to give the acetoxymethyl ester (XI) A. Bromomethyl acetate is used in a proportion of about 1 to 4 moles, preferably about 1 to 1.5 moles, per mole of compound (X) A. This reaction is conducted in an inert organic solvent such as dimethylformamide, dimethylacetamide, acetonitrile, etc., preferably dimethylacetamide or the like. The reaction temperature is about -30°C to 50°C and preferably about -20°C to 35°C. The reaction time is about 10 minutes to 5 hours and preferably about 30 minutes to 3 hours.

In the above processes from the compound (IV) A to the compound (Xi) A, the compounds (V) A to (XI) A thus produced can be separated and purified by the conventional procedures such as concentration, extraction, washing, chromatography, crystallization and recrystallization but can be used in the reaction mixture without isolation as the starting material for the next step.

The penem compound (I) obtained by the process of this aspect of the invention or the penem ester compound synthesized by using it as a synthetic intermediate, inclusive of pharmacologically acceptable salts thereof, have excellent antibacterial activity and can be used with great advantage and safety as antimicrobial agents in the therapy and prevention of bacterial infections which may be caused by a variety of pathogenic bacteria. For use as such an antibacterial drug, the compound can be administered in a variety of pharmaceutical dosage forms (tablets, granules, injections, etc.) each containing an effective amount thereof to man and other mammals (e.g. mouse, dog, cat, etc.) with great safety. For oral administration, the compound can be processed into the tablet or capsule form by the conventional procedures. Such dosage forms may further contain diluents such as lactose, glucose, sucrose, mannitol, sorbitol, cellulose, glycine, etc., lubricants such as silica, talc, stearic acid or its salt, polyethylene glycol, etc., binders such as aluminum magnesium silicate, starch, gelatin, gums, cellulose derivatives, polyvinylpyrrolidone, etc., disintegrators such as starch, alginic acid or its salt, etc., such other additives as colors, perfumes, sweeteners and so on. The dosage can be liberally selected according to the patient's age, body weight and clinical condition, the specific dosage form, the doctor's diagnosis and other factors. Generally speaking, however, the dose range of about 50 to 1,000 mg per day can be used as the standard regimen for adults (body weight 50 kg) and such a dosage can be generally administered, orally, parenterally or locally, once a day or in a few divided doses a day.

### B. Production of the Intermediates (II) A

This aspect of the present invention relates to a commercially useful process for producing an intermediate (II) A for the synthesis of penem compound (I) or a salt thereof having antibacterial activity and of value as medicines.

Having broad antibacterial spectra, penem antibiotics (I) or salts thereof have gathered attention and a variety of synthetic processes for such penems have been developed. There are known several processes for the production of a compound of the formula: 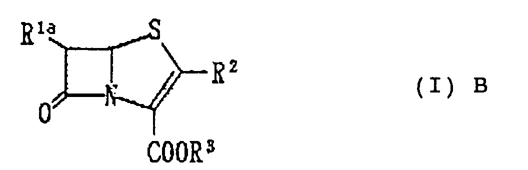
wherein R^{1a} represents a hydroxyalkyl group which may be protected, R² represents an organic group and R³ represents a carboxy-protecting group, which includes the intermediate (II) A of penem antibiotics. As such a process, EP-A-199 675 describes a process for producing a compound of the formula: 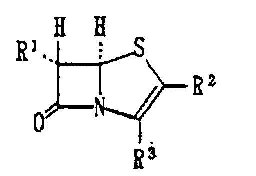
wherein R¹ represents a lower alkyl group substituted by hydroxy or protected hydroxy; R² represents a 5-membered monocyclic heteroaryl residue bound to the penem residue through a ring carbon atom and containing 1 or 2 ring nitrogen atoms or, alternatively, 1 ring nitrogen atom and 1 ring oxygen or ring sulfur atom; and R³ represents a carboxyl group or a functionally derivatized carboxyl group, which comprises reacting a compound of the formula: 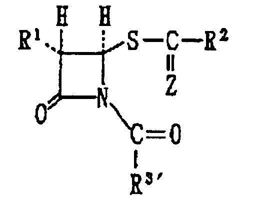
wherein R³' represents a protected carboxyl group, and the other symbols are as defined above, with an organic trivalent phosphorus compound in an inert solvent, e.g. an aromatic hydrocarbon such as benzene, toluene, etc., an ether such as dioxane, tetrahydrofuran, etc. or a halogenated hydrocarbon such as methylene chloride, chloroform, etc., at a temperature of about 20°C to 140°C and optionally carrying out any, some or all of the following steps, viz. converting the protected functional group of the resulting compound to the free functional group, converting the carboxyl group R³ of the resulting compound to a biodegradable esterified carboxyl group, converting the residue R² of the resulting compound to a different residue R², converting the resulting compound carrying a salt moiety to a free compound or a different salt, and/or resolving the resulting mixture of isomers into individual isomers.

U.S. Patent 4,826,832 describes a process for producing a compound of the formula: 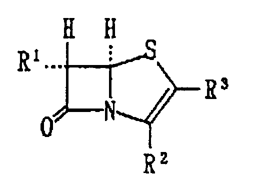
wherein R² represents a carboxyl group or a functionally modified carboxyl group and the other symbols are as defined below, which comprises subjecting a compound of the formula: 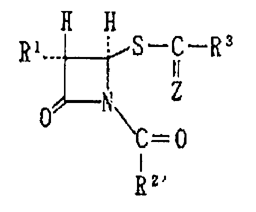
wherein R¹ represents a lower alkyl group substituted by hydroxy or protected hydroxy; R²' represents a protected carboxyl group; R³ represents a 3-pyridyl group which may be substituted or a 4-pyridyl group which may be substituted; and Z represents oxygen or sulfur, to conversion reaction using an organic trivalent phosphorus compound in an inert solvent, e.g. an aromatic hydrocarbon such as benzene, toluene, etc., an ether such as dioxane, tetrahydrofuran, etc., or a halogenated hydrocarbon such as methylene chloride, chloroform, etc., at a temperature of about 20°C to 140°C, preferably about 80°C to 120°C and optionally carrying out any, some or all of the following steps, viz. converting the protected functional group of the resulting compound to the free functional group, converting the free carboxyl group R²' of the resulting compound to a biologically cleavable esterified carboxyl group R², converting the group R³ of the resulting compound to a different group R³ and/or converting the resulting compound having a salt-forming group to a salt or converting the resulting salt to the free compound or a different salt.

Chemical Pharmaceutical Bulletin 31(2), 768-771 (1983) describes a process for producing a compound of the formula: 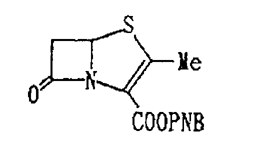
wherein Me stands for a methyl group; PNB stands for p-nitrobenzyl, which comprises reacting a compound of the formula: 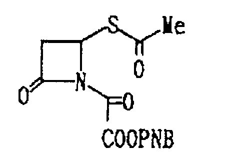
wherein the symbols are as defined above, with trimethyl phosphite at 65°C to provide a compound of the formula: 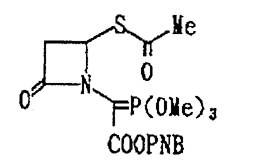
wherein the symbols have the meanings defined above, and heating the same compound in toluene at 105° for 20 hours.

Tetrahedron Letters 25, No. 22, 2395-2398 (1984) describes a process for producing a compound of the formula: 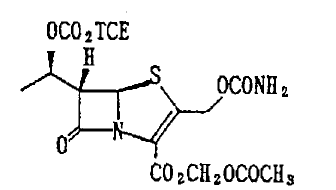
wherein TCE stands for a trichloroethyl group, which comprises reacting a compound of the formula: 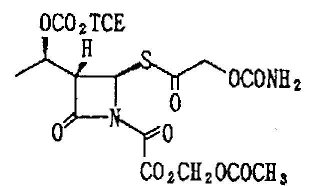
wherein the symbol has the meaning defined above, with triethyl phosphite and refluxing the reaction product in xylene.

Furthermore, GB 2042514 and GB 2042515 describe a process for producing a compound of the formula: 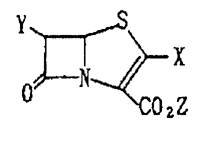
wherein Z represents hydrogen or an easily cleavable ester protective group and the other symbols are as defined below, which comprises subjecting a compound of the formula: 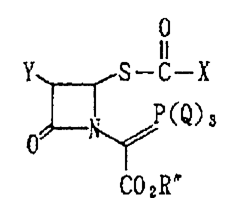
wherein Q represents a phenyl group or a (lower)alkyl group; R'' represents an easily removable ester residue; X represents a member selected from among hydroxy, aliphatic groups, etc.; and Y represents a member selected from among hydrogen, aliphatic groups, etc., to cyclization reaction in an inert organic solvent at a temperature ranging from just over room temperature to the reflux temperature of the solvent, removing the easily removable ester residue and, optionally, the other protective group by the per se known procedure and, where Y is hydrogen and if desired, treating the reaction product with an electrophilic agent in the presence of a strong base in an inert solvent to provide a desired product in which Y is not hydrogen. Incidentally, unlike the earlier-mentioned known processes, this process involves the use of a phosphine in lieu of a phosphite.

However, these known processes are disadvantageous in that the cyclization step is time-consuming, a high reaction temperature is required, unwanted byproducts are formed in large amounts, the product is of low quality and the product yield is low. Therefore, the processes are not efficient enough for the production of the objective compound (I) B. It is for these reasons that the development of an advantageous mass production process has been much awaited.

For overcoming the above drawbacks of the prior art, after an intensive research, it was discovered surprisingly that when a compound of formula: 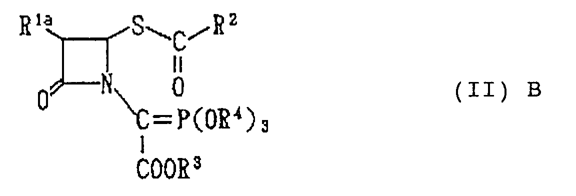
wherein R⁴ represents an alkyl group or an aryl group and the other symbols are as defined above in the formula (I) B, is heated in an alcoholic solvent, the desired reaction proceeds fast at a low temperature with little byproduct formation to provide compound (I) B in high purity and good yield. The aspect of the present invention has been developed on the basis of the above finding.

The aspect of the present invention includes:
(1) a process for producing compound (I) B, especially the compound (II) A which in the formula (I) B, R^{1a} is 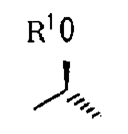 (R¹ is as defined above in (II) A) and R³ is -CH₂CH=CH₂, characterized in that compound (II) B, including a compound which in the formula (II) B, R^{1a} is 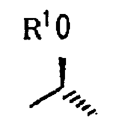 (R¹ is as defined above) and R³ is -CH₂CH=CH₂ as represented by the formula: 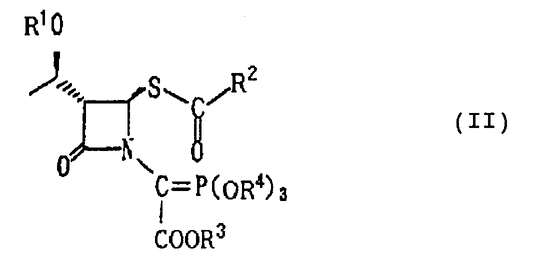 wherein all the symbols have the same meanings defined above, is heated in an alcoholic solvent;
(2) a process according to (1) wherein said solvent is a primary alcohol of not more than 8 carbon atoms;
(3) a process according to (1) further characterized in that heating is performed at 50°C to 90°C;
(4) a process according to (1) wherein R^{1a} is a 1-hydroxyethyl group which may be protected;
(5) a process according to (1) wherein R² is a group of the formula -W-R²' wherein W represents a bond, N, O or S and R²' is a hydrocarbon group which may be substituted or a heterocyclic group which may be substituted;
(6) a process according to (5) wherein said hydrocarbon group is (i) a saturated or unsaturated C₁₋₆ aliphatic group, (ii) a C₃₋₈ alicyclic hydrocarbon group or (iii) a C₄₋₁₄ aromatic hydrocarbon group;
(7) a process according to (5) wherein said heterocyclic group is a 5- or 6-membered heterocyclic group containing 1 to 3 nitrogen, sulfur and/or oxygen atoms;
(8) a process according to (1) wherein R² is a pyridyl group which may be substituted;
(9) a process according to (1) wherein R⁴ is an alkyl group; and
(10) a process according to (1) wherein R⁴ is an ethyl group.

Referring to the above formulas, R^{1a} represents a hydroxyalkyl group which may be protected. The hydroxyalkyl group thus includes hydroxyalkyl groups containing 1 to 6 carbon atoms such as hydroxymethyl, 1-hydroxyethyl, 2-hydroxyethyl, 1-hydroxypropyl, 2-hydroxypropyl, 3-hydroxypropyl, 1-hydroxy-1-methylethyl, 1-hydroxybutyl, 2-hydroxybutyl, 3-hydroxybutyl, 4-hydroxybutyl, 1-hydroxy-1-methylpropyl, 1-hydroxy-2-methylpropyl and so on. Preferred are C₁₋₄ alkyl groups substituted by hydroxyl in position-1, such as hydroxymethyl, 1-hydroxyethyl and so on. Still more preferred is 1-hydroxyethyl or the like.

The protective group for said hydroxyalkyl group includes those groups which are generally used in peptide chemistry and β-lactam chemistry, e.g. C₁₋₇ acyl groups which may be mono- to tri-substituted by halogen (e.g. chloro, bromo, fluoro, etc.), nitro, etc. such as formyl, acetyl, chloroacetyl, dichloroacetyl, trichloroacetyl, trifluoroacetyl, propionyl, allyloxyglyoxyloyl, 4-nitrobenzoyl, 2-nitrobenzoyl, etc.; C₇₋₁₉ aralkyl groups which may be mono- to tri-substituted by C₁₋₄ alkoxy (e.g. methoxy, ethoxy, etc.), nitro, etc., such as benzyl, 4-methoxybenzyl, 2-nitrobenzyl, 4-nitrobenzyl, diphenylmethyl, triphenylmethyl, etc.; C₁₋₆ alkoxy-carbonyl groups which may be mono- to tri-substituted by C₁₋₄ alkoxy (e.g. methoxy, ethoxy, etc.), halogen (e.g. chloro, bromo, etc.), tri-C₁₋₄ alkylsilyl (e.g. trimethylsilyl, etc.), etc., such as methoxycarbonyl, ethoxycarbonyl, t-butoxycarbonyl, 2,2,2-trichloroethoxycarbonyl, 2-trimethylsilylethoxycarbonyl, etc.; C₇₋₁₉ aralkyloxycarbonyl groups which may be substituted by C₁₋₄ alkoxy (e.g. methoxy, ethoxy, etc.), nitro, etc., such as benzyloxycarbonyl, 4-methoxybenzyloxycarbonyl, 3,4-dimethoxybenzyloxycarbonyl, 2-nitrobenzyloxycarbonyl, 4-nitrobenzyloxycarbonyl, etc.; C₂₋₆ alkenyloxy-carbonyl groups such as vinyloxycarbonyl, allyloxycarbonyl, etc.; substituted methyl groups as mono- to tri-substituted by C₁₋₄ alkoxy (e.g. methoxy, ethoxy, etc.), which may, in turn, be substituted by halogen (e.g. chloro, bromo, fluoro, etc.), etc., such as methoxymethyl, t-butoxymethyl, 2-methoxyethoxymethyl, 2,2,2-trichloroethoxymethyl, etc.; substituted ethyl groups as mono- to tri-substituted by C₁₋₄ alkoxy (e.g. methoxy, ethoxy, etc.), halogen (e.g. chloro, bromo, fluoro, etc.), etc., such as 1-ethoxyethyl, 1-methyl-1-methoxyethyl, 2,2,2-trichloroethyl, etc.; tri-C₁₋₄ alkylsilyl groups such as trimethylsilyl, triethylsilyl, dimethylisopropylsilyl, t-butyldimethylsilyl, triisopropylsilyl, etc.; and diphenyl-C₁₋₄ alkylsilyl groups such as diphenylmethylsilyl, diphenylethylsilyl, etc. Preferred are C₇₋₁₉ aralkyloxy-carbonyl (e.g. benzyloxycarbonyl), C₂₋₆ alkenyloxy-carbonyl (e.g. vinyloxycarbonyl), tri-C₁₋₄ alkylsilyl (e.g. trimethylsilyl), etc. More preferred are tri-C₁₋₄ alkylsilyl groups, etc. and the most advantageous is tert-butyldimethylsilyl, etc.

The preferred species of R^{1a} includes, among others, C₁₋₄ alkyl groups substituted by hydroxyl in 1-position, which may optionally be protected by tri-C₁₋₄ alkyl silyl, such as hydroxymethyl, 1-hydroxyethyl, 1-(tert-butyldimethylsilyloxy)ethyl, etc. More preferred are 1-hydroxyethyl which may be protected by tri-C₁₋₄ alkylsilyl, such as 1-hydroxyethyl, 1-(tert-butyldimethylsilyloxy)ethyl, etc. and the most useful are 1-hydroxyethyl, 1-(tert-butyldimethyloxy)ethyl, etc.

Referring, further, to the above formulas, R² represents an organic group. The organic group is a group of the formula -W-R²' wherein W represents a bond, N, O or S and R²' represents a hydrocarbon group which may be substituted or a heterocyclic group which may be substituted.

The hydrocarbon moiety of this optionally substituted hydrocarbon group R²' includes, among others, (1) saturated or unsaturated C₁₋₆ aliphatic hydrocarbon groups, (2) C₃₋₈ alicyclic hydrocarbon groups and (3) C₆₋ ₁₄ aromatic hydrocarbon groups. Preferred are C₁₋₆ alkyl groups, C₂₋₆ alkenyl groups, C₂₋₆ alkynyl groups, C₃₋₈ cycloalkyl groups, C₆₋₁₄ aryl groups, etc. Still more preferred are C₁₋₆ alkyl groups, C₆₋₁₄ aryl groups, etc.

The C₁₋₆ alkyl groups mentioned above include methyl, ethyl, propyl, isopropyl, butyl, isobutyl, sec-butyl, tert-butyl, etc. Preferably, C₁₋₄ alkyl groups such as methyl, ethyl, propyl, isopropyl, etc. are employed.

The C₂₋₆ alkenyl groups include ethenyl, propenyl, isopropenyl, butenyl, isobutenyl, sec-butenyl, etc. Preferably, C₂₋₄ alkenyl groups such as ethenyl, propenyl, isopropenyl, etc. are employed.

The C₂₋₆ alkynyl groups include ethynyl, propynyl, isopropynyl, butynyl, isobutynyl, sec-butynyl, etc. Preferred are C₂₋₄ alkynyl groups such as ethynyl, propynyl, isopropynyl, etc.

The C₃₋₈ cycloalkyl groups include cyclopropyl, cyclobutyl, cyclopentyl, cyclohexyl, etc. Preferred are C₃₋₆ cycloalkyl groups such as cyclopropyl, cyclobutyl, etc.

The C₆₋₁₄ aryl groups include phenyl, naphthyl, anthryl, phenanthryl, etc. Preferred are C₆₋₁₀ aryl groups such as phenyl and naphthyl.

The heterocyclic moiety of the optionally substituted heterocyclic group R²' includes, among others, 5- or 6-membered heterocyclic groups containing 1 to 3 nitrogen, sulfur and/or oxygen atoms in addition to carbon atoms. Preferred are 5-membered heterocyclic groups containing one nitrogen, sulfur or oxygen atom, such as pyrrole, pyrroline, thienyl, furyl, etc.; nitrogen-containing 6-membered heterocyclic groups such as 6-membered heterocyclic groups containing 1 or 2 nitrogen atoms in addition to carbon atoms, e.g. pyridyl, pyrimidyl, pyridazinyl, etc. and nitrogen-containing 5-membered heterocyclic groups such as 5-membered heterocyclic groups containing 2 nitrogen atoms in addition to carbon atoms, e.g. pyrazolyl, imidazolyl, etc., 5-membered heterocyclic groups containing 1 nitrogen atom and 1 sulfur atom in addition to carbon atoms, e.g. thiazolyl etc., 5-membered heterocyclic groups containing 2 nitrogen atoms and 1 sulfur atom in addition to carbon atoms, e.g. 1,2,3-thiadiazolyl, 1,2,4-thiadiazolyl, 1,3,4-thiadiazolyl, 1,2,5-thiadiazolyl, etc., 5-membered heterocyclic groups containing 2 nitrogen atoms and 1 oxygen atom in addition to carbon atoms, e.g. 1,2,3-oxadiazolyl, 1,2,4-oxadiazolyl, 1,3,4-oxadiazolyl, 1,2,5-oxadiazolyl, etc., 5-membered heterocyclic groups containing 3 nitrogen atoms in addition to carbon atoms, e.g. 1,2,5-triazolyl, 1,2,4-triazolyl, etc., and 5-membered groups containing 4 nitrogen atoms in addition to a carbon atom, e.g. 1H-tetrazolyl, 2H-tetrazolyl, etc. More preferred are 5-membered heterocyclic groups containing one nitrogen, sulfur or oxygen atom in addition to carbon atoms and 6-membered heterocyclic groups containing 1 or 2 nitrogen atoms in addition to carbon atoms. Particularly preferred are thienyl, furyl, pyridyl, etc.

The substituents on the above optionally substituted hydrocarbon group and optionally substituted heterocyclic group, represented by R²', include, among others, (1) hydroxy, (2) oxo, (3) mercapto, (4) halogen, (5) carboxy, (6) carbamoyl, (7) carbamoyloxy, (8) alkoxy, (9) alkoxycarbonyl, (10) alkylthio, (11) alkanoyl, (12) alkanoyloxy, (13) amino optionally substituted by one or two of alkyl and alkanoyl, where the substituents may be the same or different, (14) cycloalkyl, (15) aryl and (16) heterocyclic groups, and where the hydrocarbon group is an alicyclic hydrocarbon group or an aromatic hydrocarbon group, (17) alkyl groups optionally substituted by 1 or 2 members, which may be the same or different, of hydroxy, halogen, amino and carboxy. The number of the substitutent is one to five, preferably one or two in the possible position(s).

The halogen mentioned above includes chloro, fluoro, bromo and iodo and may preferably be chloro or fluoro.

The alkoxy group includes C₁₋₆ alkoxy groups such as methoxy, ethoxy, propoxy, isopropoxy, butoxy, isobutoxy, sec-butoxy, tert-butoxy, etc. Preferred are C₁₋₄ alkoxy groups such as methoxy, ethoxy, propoxy, isopropoxy, etc.

The alkoxy moiety of the alkoxycarbonyl group includes the alkoxy groups mentioned above.

The alkyl moiety of the alkylthio group includes the alkyl groups mentioned above for the hydrocarbon of R²'.

The alkanoyl group includes C₁₋₆ alkanoyl groups such as acetoxy, pivaloyloxy, etc. Preferred are C₁₋₄ alkanoyl groups such as acetoxy, etc.

The alkanoyl moiety of the alkanoyloxy group includes the alkanoyl groups mentioned above.

The alkyl or alkanoyl moiety of the optionally substituted amino group as substituted by 1 or 2 members, which may be the same or different, of alkyl and alkanoyl groups includes the above-mentioned groups. Examples of the optionally substituted amino group include C₁₋₆ alkylamino, di-C₁₋₆ alkylamino and C₁₋₆ alkanoylamino groups such as methylamino, ethylamino, dimethylamino, diethylamino, methylethylamino, acetoxyamino, etc. Preferred are C₁₋₄ alkylamino, di-C₁₋ ₄ alkylamino and C₁₋₄ alkanoylamino groups such as methylamino, ethylamino, dimethylamino, diethylamino, acetoxyamino, etc.

The cycloalkyl, aryl and heterocyclic groups include the same groups as mentioned previously for R²' representing a hydrocarbon or heterocyclic group.

The halogen of the optionally substituted alkyl group as substituted by 1 or 2 members, which may be the same or different species, of hydroxy, halogen, amino and carboxy includes the same halogen species as mentioned hereinbefore. Similarly, the alkyl moiety of said optionally substituted alkyl group includes the groups mentioned above for said hydrocarbon group of R²'. Examples of the optionally substituted alkyl group include methyl, ethyl, hydroxymethyl, hydroxyethyl, hydroxypropyl, chloromethyl, chloroethyl, fluoromethyl, fluoroethyl, aminomethyl, aminoethyl, carboxymethyl, carboxyethyl, etc. Preferred are methyl, ethyl, hydroxymethyl, fluoromethyl, amino-methyl, carboxymethyl, etc.

The preferred substituent on the hydrocarbon group which may be substituted or on the heterocyclic group which may be substituted includes, among others, (a) hydroxy, (b) oxo, (c) halogen, (d) carboxy, (e) carbamoyl, (f) carbamoyloxy, (g) C₁₋₄ alkoxy, (h) amino, (i) C₁₋₆ alkyl which may be substituted by 1 or 2 identical or different members of hydroxy, halogen, amino and/or carboxy, etc. Still more preferred are (a) hydroxy, (b) oxo, (e) carbamoyl, (f) carbamoyloxy, (g) C₁₋₄ alkoxy, (h) amino, (i) C₁₋₄ alkyl, etc.

The preferred species of R² include, among others, (i) C₁₋₆ alkyl groups which may be substituted by a carbamoyl, carbamoyloxy or C₁₋₄ alkoxy, (ii) C₆₋₁₀ aryl groups which may be substituted by a carbamoyl, carbamoyloxy or C₁₋₄ alkoxy; (iii) nitrogen-, sulfur- or oxygen-containing 5-membered heterocyclic groups which may be bound through N, O or S and be substituted by oxo, carbamoyl, amino or C₁₋₄ alkyl groups; (iv) 6-membered heterocyclic groups containing 1 or 2 nitrogen atoms, etc. More preferred are C₁₋₆ alkyl groups which may be substituted by a carbamoyloxy or C₁₋₄ alkoxy; C₆₋ ₁₀ aryl groups which may be substituted by carbamoyl; nitrogen-, sulfur- or oxygen-containing 5-membered heterocyclic groups which may be bound through S and be substituted by an oxo; 6-membered heterocyclic groups containing 1 or 2 nitrogen atoms, etc. Particularly preferred are C₁₋₄ alkoxy-C₁₋₄ alkyl, carbamoyloxy-C₁₋₄ alkyl, carbamoyl-C₆₋₁₀ aryl, thienylthio, S-oxidothienylthio, furyl, pyridyl, etc.

Referring to the above formulas, R³ represents a carboxy-protecting group. This group includes, among others, C₁₋₈ alkyl groups such as methyl, ethyl, propyl, isopropyl, butyl, isobutyl, sec-butyl, tert-butyl, hexyl, etc., C₁₋₆ alkyl groups substituted by 1 to 3 halogen atoms (e.g. chloro, bromo, fluoro and iodo), such as bromo-tert-butyl, trichloroethyl, etc., C₂₋₆ alkenyl groups such as vinyl, allyl, etc., C₇₋₁₄ aralkyl groups which may be mono- or di-substituted by nitro, C₁₋₄ alkoxy (e.g. methoxy, ethoxy, etc.), C₁₋₄ alkyl (e.g. methyl, ethyl, propyl, isopropyl, butyl, isobutyl, sec-butyl, tert-butyl, etc.), etc., such as benzyl, p-nitrobenzyl, o-nitrobenzyl, p-methoxybenzyl, p-tert-butylbenzyl, etc., C₁₋₄ alkanoyloxy-C₁₋₄ alkyl groups such as acetoxymethyl, propionyloxymethyl, butyryloxymethyl, isobutyryloxymethyl, valeryloxymethyl, pivaloyloxymethyl, 1-(or 2-)acetoxyethyl, 1-(or 2- or 3-)acetoxypropyl, 1-(or 2-, 3- or 4-)acetoxybutyl, 1-(or 2-)propionyloxyethyl, 1-(or 2- or 3-)propionyloxypropyl, 1-(or 2-)butyryloxyethyl, 1-(or 2-)isobutyryloxyethyl, 1-(or 2-)pivaloyloxyethyl, 1-(or 2-)hexanoyloxyethyl, isobutyryloxymethyl, 2-ethylbutyryloxymethyl, 3,3-dimethylbutyryloxymethyl, 1-(or 2-)pentanoyloxyethyl, etc., C₁₋₄ alkanesulfonyl-C₁₋₄ alkyl groups such as 2-mesylethyl, etc., C₁₋₄ alkoxycarbonyloxy-C₁₋₄ alkyl groups such as methoxycarbonyloxymethyl, ethoxycarbonyloxymethyl, propoxycarbonyloxymethyl, tert-butoxycarbonyloxymethyl, 1-(or 2-)methoxycarbonyloxyethyl, 1-(or 2-)ethoxycarbonyloxyethyl, 1-(or 2-)isopropoxycarbonyloxyethyl, etc., tri-C₁₋₄ alkylsilyl groups such as tert-butyldimethylsilyl, trimethylsilyl, etc., C₂₋₆ alkenyl groups such as allyl, methallyl, etc., C₁₋₄ alkoxymethyl groups such as methoxymethyl, ethoxymethyl, propoxymethyl, isopropoxymethyl, etc., C₁₋₄ alkylthio-C₁₋₄ alkyl groups such as 2-(methylthio)ethyl, etc., 3-methyl-2-butenyl, 5-indanyl, 3-phthalidyl and so on. Preferred are C₇₋₁₄ aralkyl groups which may be mono- or di-substituted by C₂₋₆ alkenyl, nitro, C₁₋₄ alkyl or the like. Particularly preferred are allyl, p-nitrobenzyl and the like.

In the compound (II) B to be used in the process of this aspect of the invention as well as in the resultant compound (I) B, the hydroxyalkyl-protecting group and the carboxy-protecting group may be the same or different.

Referring, further, to the above formulas, R⁴ represents an alkyl group or an aryl group. The alkyl group includes C₁₋₆ alkyl groups such as methyl, ethyl, propyl, isopropyl, butyl, isobutyl, sec-butyl, tert-butyl, etc. and is preferably a C₁₋₄ alkyl group such as methyl, ethyl, propyl, isopropyl, etc. The aryl group includes C₆₋₁₄ aryl groups such as phenyl, naphthyl, anthryl, phenanthryl, etc. and is preferably a C₆₋₁₀ aryl group such as phenyl, naphthyl, etc.

In accordance with the process of this aspect of the invention, compound (II) B is heated in an alcoholic solvent to provide compound (I) B.

The alcoholic solvent which can be employed includes, among others, C₁₋₈ aliphatic saturated alcohols such as methyl alcohol, ethyl alcohol, 1-propyl alcohol, 1-butyl alcohol, 2-methyl-1-propyl alcohol, 1-pentyl alcohol, 1-octyl alcohol, 2-propyl alcohol, 2-butyl alcohol, 2-pentyl alcohol, 3-pentyl alcohol, 2-methyl-2-propyl alcohol, 2-methyl-2-butyl alcohol, etc., C₂₋₆ aliphatic unsaturated alcohols such as allyl alcohol etc., C₃₋₈ alicyclic alcohols such as cyclopentyl alcohol, cyclohexyl alcohol, etc., and C₆₋₁₀ aromatic alcohols such as benzyl alcohol, etc. These alcohols may be substituted by C₁₋₄ alkoxy (e.g. methoxy, ethoxy, propoxy, isopropoxy, etc.), ester residue (e.g. may form an ester with a C₁₋₄ alkyl group, etc.), nitro, halogen (e.g. chloro, bromo, iodo, fluoro, etc.), etc., for example as it is the case with 2-methoxyethyl alcohol, ethyl glycolate, nitromethyl alcohol, 2-nitroethyl alcohol, 2,2,2-trichloroethyl alcohol, 2,2,2-trifluoroethyl alcohol, 1,1,1,3,3,3-hexafluoro-2-propyl alcohol, etc.

The preferred species of said alcoholic solvent includes, among others, C₁₋₈ primary aliphatic saturated alcohols such as methyl alcohol, ethyl alcohol, 1-propyl alcohol, 1-butyl alcohol, 2-methyl-1-propyl alcohol, 1-pentyl alcohol, 1-octyl alcohol, 2-methyl-2-propyl alcohol, 2-methyl-2-butyl alcohol, etc., C₂₋₆ aliphatic unsaturated primary alcohols such as allyl alcohol, etc. and C₆₋₁₀ aromatic primary alcohols such as benzyl alcohol, etc. These primary alcohols may be substituted by C₁₋₄ alkoxy (e.g. methoxy, ethoxy, propoxy, isopropoxy, etc.), nitro, halogen (e.g. chloro, bromo, iodo and fluoro), etc., for example as it is the case with 2-methoxyethyl alcohol, nitromethyl alcohol, 2-nitroethyl alcohol, 2,2,2-trichloroethyl alcohol, 2,2,2-trifluoroethyl alcohol, etc. but in consideration of the subsequent step, e.g. concentration, it is preferable to employ an unsubstituted primary alcohol.

Particularly preferred species of said alcoholic solvent are C₁₋₆ aliphatic saturated primary alcohols such as methyl alcohol, ethyl alcohol, 1-propyl alcohol, 1-butyl alcohol, 2-methyl-1-propyl alcohol, etc. and the most preferred alcoholic solvents are C₂₋₄ aliphatic saturated primary alcohols such as ethyl alcohol, 1-propyl alcohol, 1-butyl alcohol, 2-methyl-1-propyl alcohol, etc.

The concentration of compound (II) B in said solvent is about 1 to 50% (w/w) and preferably about 5 to 30% (w/w). The heating temperature may range from about 30°C to the reflux temperature of the solvent and is preferably about 50 to 90°C. The reaction time is about 30 minutes to 5 hours and preferably about 1 to 3 hours. After completion of the reaction, the yield of product compound (I) B can be estimated by high performance liquid chromatography, among other techniques. The compound (I) B thus produced can be separated and purified by the conventional isolation procedures such as concentration, extraction, washing, chromatography, crystallization, recrystallization, etc. but the reaction product can be directly utilized as a starting material for the synthesis of the next step.

The starting compound (II) B can be provided by reacting a compound of formula: 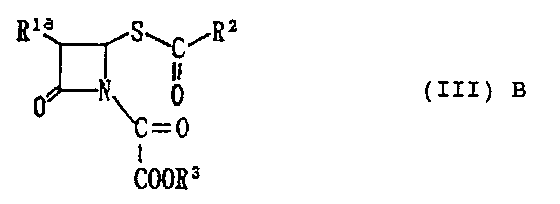
wherein the symbols have the same meanings as defined hereinbefore, including a compound wherein R^{1a} is 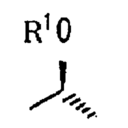
(R¹ is as defined above) and R³ is -CH₂CH=CH₂ as represented by the formula: 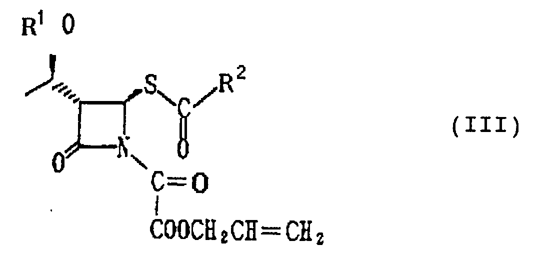
wherein all the symbols have the same meanings defined above, and a compound wherein R^{1a} is 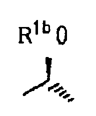
(R^{1b} is a sillyl group) and R³ is -CH₂CH=CH₂ as represented by the formula: 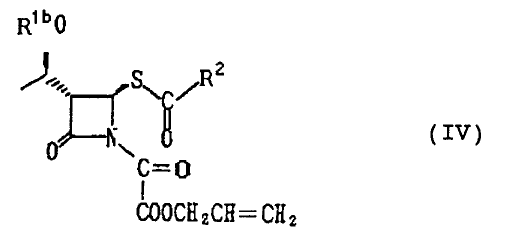
wherein all the symbols have the same meanings above, with an organic trivalent phosphorus compound. The organic trivalent phosphorus compound that can be employed includes, among others, tri-C₁₋₆ alkyl phosphites such as trimethyl phosphite, triethyl phosphite, tripropyl phosphite, tributyl phosphite, etc. and tri-C₆₋₁₀ aryl phosphites such as triphenyl phosphite etc. Preferred are tri-C₂₋₄ alkyl phosphites such as triethyl phosphite, tributyl phosphite, etc. The proportion of the organic trivalent phosphorus compound is generally about 2 to 50 moles and preferably about 5 to 30 moles per mole of compound (III) B.

This reaction can be conducted in the absence of a solvent or in an inert organic solvent (e.g. dichloromethane, xylene, toluene, dioxane, etc.). The reaction temperature is about 20 to 80°C and preferably about 40 to 60°C. The reaction time is about 0.5 to 10 hours and preferably about 1 to 4 hours.

The compound (III) B can be synthesized by the processes described or implied in EP-A-199675, U.S. Patent 4,826,832, Chemical Pharmaceutical Bulletin 31(2), 768-771 (1983), Tetrahedron Letters 25, No. 22, 2395-2398 (1984) and so on.

As the resulting compound (II) B, the residue available on concentration of the reaction mixture under reduced pressure, or the residue obtainable by dissolving said residue in a suitable non-aqueous inert organic solvent (e.g n-hexane, dichloromethane, etc.), washing it with water and subjecting the thus-purified preparation to drying and concentration can be also used in this aspect. As a further alternative, the reaction mixture containing compound (II) B can be dissolved in an alcoholic solvent for use in the process of this aspect of the invention.

The compound (I) B thus obtained can be used with advantage, for example as a synthetic intermediate for the production of penem antibiotics including the compound (II) A.

By way of illustration, the compound of formula: 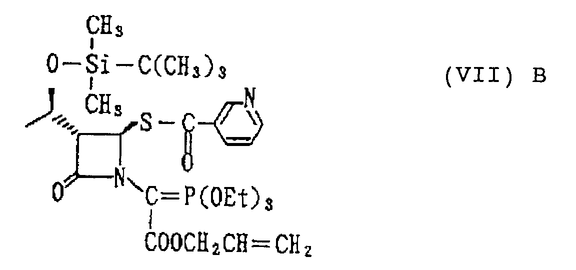
, which is subsumed in the category of starting compound (II) B for this aspect of the invention, can be synthesized from (3R,4R)-4-acetoxy-3-[(R)-1-(tert-butyldimethylsilyloxy)ethyl]-2-azetidinone of the formula: 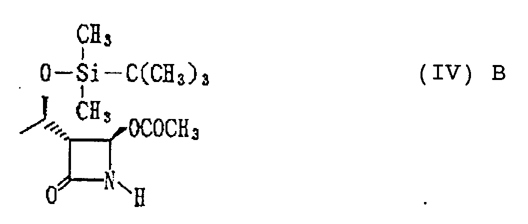
, which is an intermediate of penem antibiotics, in accordance with the following reaction schema. 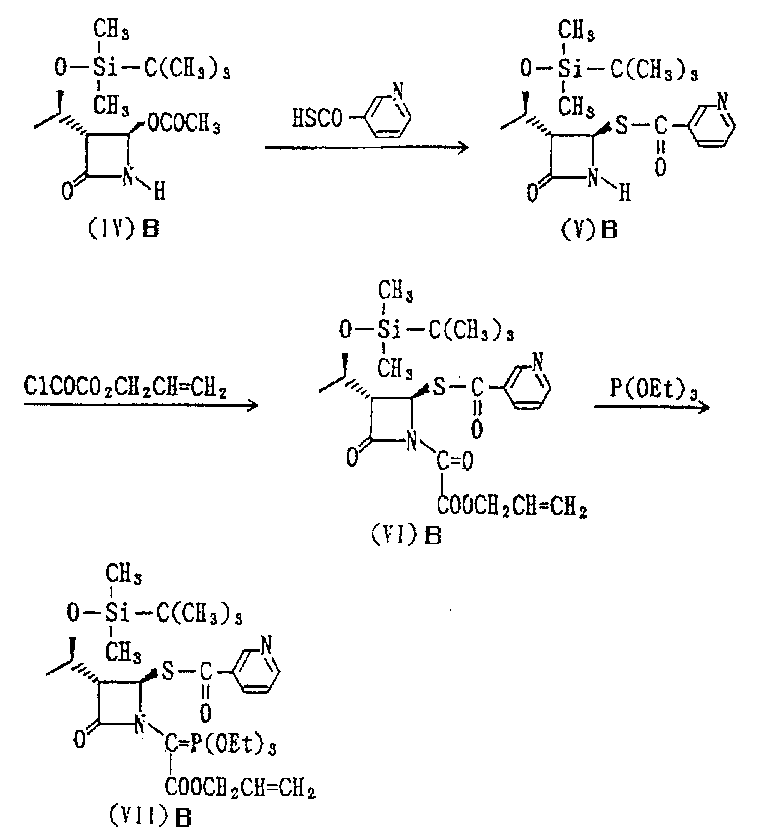
Thus, compound (IV) B is reacted with thionicotinic acid to substitute an acetoxy group with a nicotinoylthio group, giving rise to compound (V) B. This reaction is carried out in a binary solvent system comprising an inert organic solvent (e.g. dichloromethane, ethyl acetate, toluene, etc.) and water, preferably dichloromethane-water. Thionicotinic acid is used in a proportion of about 1 to 3 moles, preferably about 1 to 1.5 moles, per mole of compound (IV) B. The reaction temperature is about 0°C to 40°C and preferably about 10°C to 30°C. The reaction time is about 0.5 to 30 hours and preferably about 1 to 24 hours.

Compound (V) B is converted to compound (VI) B by introducing an allyloxyglyoxyloyl group through reaction with allyloxyoxalyl chloride in the presence of a tri-lower-alkylamine, such as triethylamine, N,N-diisopropylethylamine, etc., preferably triethylamine or N,N-diisopropylethylamine. The allyloxyoxalyl chloride is used in a proportion of about 1 to 2 moles, preferably about 1 to 1.5 moles, per mole of compound (V) B. This reaction is carried out in an inert organic solvent (e.g. dichloromethane, ethyl acetate, toluene, etc.), preferably in dichloromethane. The reaction temperature is about -40°C to 30°C and preferably about -20°C to 20°C. The reaction time is not more than 2 hours and preferably 3 to 90 minutes.

The resulting compound (VI) B is further reacted with triethyl phosphite to give compound (VII) B. This reaction may be carried out in the absence of a solvent or in an inert organic solvent (e.g. dichloromethane, xylene, toluene, dioxane, etc.). Triethyl phosphite is used in a proportion of generally about 2 to 50 moles and preferably about 5 to 30 moles per mole of compound (VI) B. The reaction temperature is about 20 to 80°C and preferably about 40 to 60°C. The reaction time is about 0.5 to 10 hours and preferably about 1 to 4 hours.

Compound (VII) B is heated in 2-methyl-1-propyl alcohol in accordance with this aspect of the invention to produce a compound of the formula: 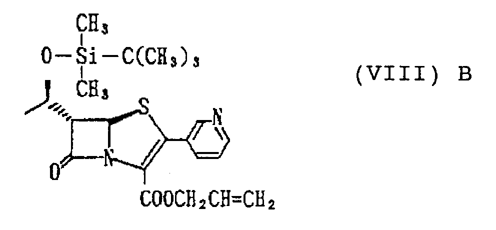
This compound (VIII) B can be further reacted as shown in the following reaction shema to provide acetoxymethyl (5R, 6S)-6-[(R)-1-hydroxyethyl]-2-(3-pyridyl)-2-penem-3-carboxylate which exhibits a particularly outstanding antibacterial action after oral administration, as represented by the formula: 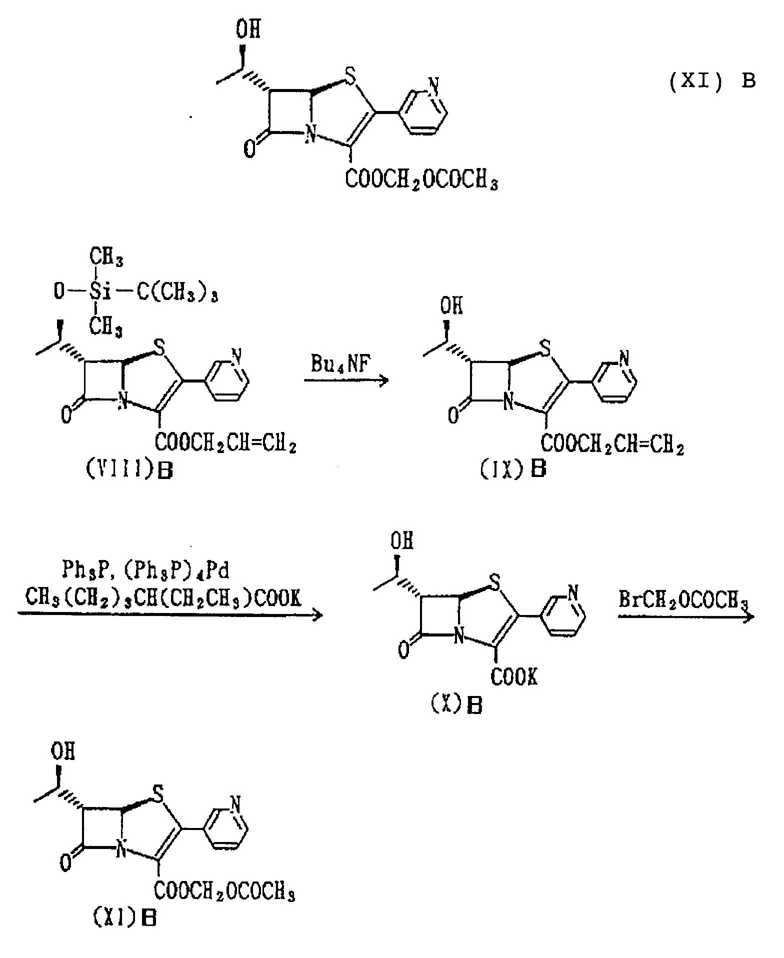
Thus, compound (VIII) B is reacted with tetrabutylammonium fluoride in the presence of acetic acid to remove the hydroxy-protecting tert-butyldimethylsilyl group and thereby provide compound (IX) B.

This reaction is conducted in an inert organic solvent such as tetrahydrofuran, dichloromethane, acetonitrile, etc., preferably tetrahydrofuran. The tetrabutylammonium fluoride is used in a proportion of 1 to 20 moles, preferably 2 to 10 moles, per mole of compound (VIII) B. The reaction temperature is 0 to 50°C and preferably 15 to 30°C. The reaction time is 30 minutes to 40 hours and preferably 5 to 30 hours.

Compound (IX) B is then treated with tetrakis(triphenylphosphine)palladium and potassium 2-ethylhexanoate to remove the carboxy-protecting allyl group and thereby provide a penem potassium salt (X) B.

Tetrakis(triphenylphosphine)palladium is used in a catalytic amount, namely 0.0001 to 0.1 mole, preferably 0.01 to 0.02 mole, per mole of compound (IX) B. This reaction is preferably carried out with the addition of triphenylphosphine, the amount of which is preferably 2 to 10 moles per mole of tetrakis(triphenylphosphine)palladium. Potassium 2-ethylhexanoate is used in a proportion of 1 to 4 moles, preferably 1.05 to 2 moles, per mole of compound (IX) B.

This reaction is carried out in the presence of a solvent. The solvent is not critical in type insofar as it does not interfere with the reaction. For example, the reaction can be conducted in an inert organic solvent such as tetrahydrofuran, acetonitrile, acetone, dichloromethane, etc., preferably in tetrahydrofuran or tetrahydrofuran-dichloromethane. The reaction temperature is -20°C to 50°C and preferably 0-40°C. The reaction time is not more than 8 hours and preferably 5 minutes to 3 hours.

The compound (X) B thus obtained can be reacted with bromomethyl acetate to give the acetoxymethyl ester (XI) B.

Bromomethyl acetate is used in a proportion of about 1 to 4 moles, preferably about 1 to 1.5 moles, per mole of compound (X) B. This reaction is conducted in an inert organic solvent such as dimethylformamide, dimethylacetamide, acetonitrile, etc., preferably dimethylacetamide or the like. The reaction temperature is -30°C to 50°C and preferably -20°C to 35°C. The reaction time is 10 minutes to 5 hours and preferably 30 minutes to 3 hours.

In the above processes from the compound (IV) B to the compound (XI) B, each of the produced compounds can be separated and purified by the conventional procedures such as concentration, extraction, washing, chromatography, crystallization and recrystallization, but can be also used in the reaction mixture without isolation as the starting material for the next step.

The penem compound synthesized by using the intermediate compound (I) B obtained by the process of this aspect of the invention, inclusive of its pharmacologically acceptable salts, has excellent antibacterial activity and can be used with great advantage and safety as antimicrobial agents in the therapy and prevention of bacterial infections which may be caused by a variety of pathogenic bacteria. For use as such an antibacterial drug, the compound can be administered in a variety of pharmaceutical dosage forms (tablets, granules, injections, etc.) each containing an effective amount thereof to man and other mammalian animals (e.g. mouse, dog, cat, etc.) with great safety. For oral administration, the compound can be processed into the tablet or capsule form by the conventional procedures. Such dosage forms may further contain diluents such as lactose, glucose, sucrose, mannitol, sorbitol, cellulose, glycine, etc., lubricants such as silica, talc, stearic acid or its salt, polyethylene glycol, etc., binders such as aluminum magnesium silicate, starch, gelatin, gums, cellulose derivatives, polyvinylpyrrolidone, etc., disintegrators such as starch, alginic acid or its salt, etc. and such other additives as colors, perfumes, sweeteners and so on. The dosage can be liberally selected according to the patient's age, body weight and clinical condition, the specific dosage form, the doctor's diagnosis and other factors. Generally speaking, however, the dose range of about 50 to 1,000 mg per day can be used as the standard regimen for adults (body weight 50 kg) and such a dosage can be generally administered, orally, parenterally or locally, once a day or in a few divided doses a day.

It is to be understood that while U.S. Patent 4,508,649, U.S. Patent 4,997,829, U.S. Patent 4,782,146, etc. describe processes for producing useful antibacterial penems, the compound (I) B produced by the process of this aspect of the invention can be utilized as the starting material in such processes.

### C. Production of the Intermediates (III) wherein R¹ represents a hydrogen atom

This aspect of the invention relates to a commercially advantageous process for producing a synthetic intermediate for the production of penem compounds of value as antibacterial agents.

In the synthesis of penem compounds which can be used as antibacterials, t-butyldimethylsilyl is generally used as the protective group for the hydroxyl function of the 3-hydroxyalkyl moiety of starting azetidinoneglyoxylate compounds.

The use of an acid in the removal of the t-butyldimethylsilyl group protecting the hydroxyl function is well known. However, it is also known that any azetidinoneglyoxylate compound of the formula: 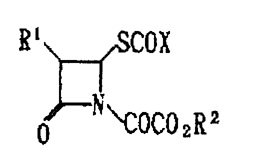
wherein R¹ represents hydroxy(lower)alkyl, etc.; R² represents alkyl, etc.; and X represents lower alkyl, etc., is ready to undergo hydrolysis, alcoholysis or hydrazinolysis to give a compound of the formula: 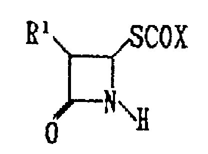
wherein the symbols have the same meanings as defined above [cf. U. S. Patent 4,692,442]. It has, therefore, been the common understanding to this day that permitting an acid to act on an azetidinoneglyoxylate precursor of penem compounds should result in cleavage of the 1-glyoxylate moiety, thus failing to provide the objective compound in which the hydroxy-protecting silyl group alone has been selectively eliminated.

Furthermore, as a technology for selective elimination of the silyl group, it has been proposed to take the procedure employing a large amount of tetrabutylammonium fluoride either before or after the cyclizing synthesis of a penem compound. Thus, U. S. Patent 5,036,063, for instance, discloses, as Example 1 of the invention, a process in which the protective silyl group is removed following penem-ring closure in the following reaction schema. 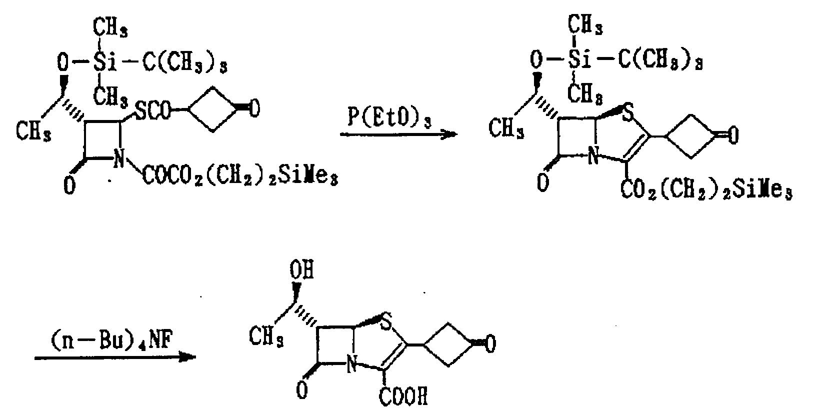
wherein Me stands for methyl, Et for ethyl and Bu for butyl.

Moreover, U. S. Patent 4,997,829 discloses a process in which the silyl group is eliminated using tetrabutylammonium fluoride prior to penem-ring closure in the following reaction schema. 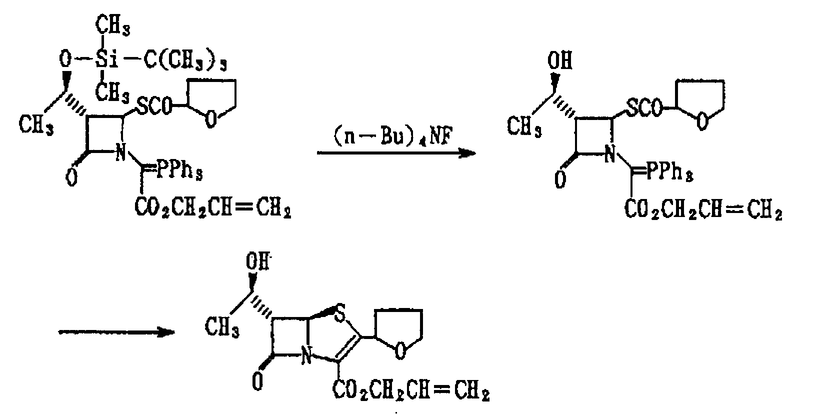
wherein Ph stands for phenyl and Bu stands for butyl.

However, tetrabutylammonium fluoride which is thus used prevalently in the elimination of the silyl group is not only expensive but so hygroscopic and labile that it calls for due circumspection in handling, for example storage in a nitrogen-purged container in the refrigerator.

There has existed, in the synthesis of penem compounds, a need for eliminating only the silyl group from an intermediate azetidinoneglyoxylate of general formula: 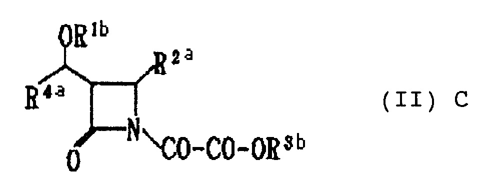
wherein R^{1b} stands for a silyl group, R^{2a} for an organic group, R^{3b} for H or a carboxy-protecting group, R^{4a} for an alkyl group, without resort to employment of said hard-to-use tetrabutylammonium fluoride to give a compound of formula: 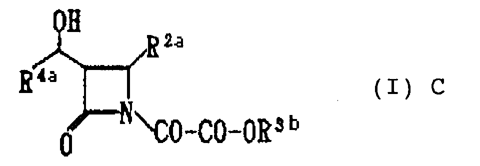
wherein the symbols have the same meanings as defined above or a salt thereof advantageously on a commercial scale and thereafter converting the latter to a penem compound.

After an extensive exploration into the production technology for compound (I) C or its salt, it was surprisingly found that permitting an acid to act on compound C, including the compound (IV) which in the formula (II) C, R^{2a} is -S-CO-R² (R² is an organic group), R^{3b} is -CH₂CH=CH₂ and R^{4a} is methyl group, results in a selective elimination of the hydroxy-protecting silyl group R^{1b} without affecting the 1-glyoxylate moiety of the azetidinone compound and that this reaction provides for the production of compound (I) C, including the compound (III) (R¹ is H) which in the formula (I) C, R^{2a} is -S-CO-R² (R² is an organic group), R^{3b} is -CH₂CH=CH₂ and R^{4a} is methyl group, or a salt thereof in high yield and high purity grade. The aspect of the present invention has been developed on the basis of the above findings.

This aspect of the invention includes therefore:
(1) a process for producing compound (I) C or a salt thereof characterized in that an acid is permitted to act on compound (II) C;
(2) the process of (1) wherein an organic solvent is used;
(3) the process of (2) wherein said organic solvent is acetonitrile;
(4) the process of (1) wherein said acid is hydrochloric acid;
(5) the process of (1) wherein R¹ is t-butyldimethylsilyl;
(6) a novel compound of the formula: 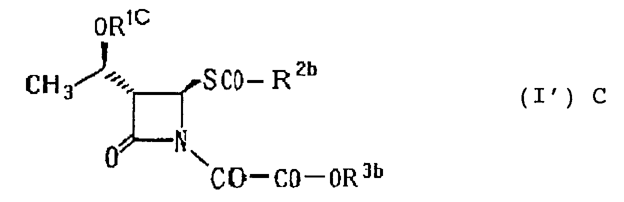 wherein R^{1c} stands for hydrogen or a silyl group; CO-R^{2b} stands for an acyl group; R^{3b} stands for hydrogen or a carboxy-protecting group, or a salt thereof; and
(7) the compound of (6) in which R^{2b} is pyridin-3-yl and R^{3b} is allyl.

Referring to the above formulas, R^{1b} represents a silyl group and R^{1c} represents a hydrogen atom or a silyl group. The silyl group includes, as preferred examples, tri-C₁₋₄ alkylsilyl groups such as trimethylsilyl, triethylsilyl, dimethylisopropylsilyl, t-butyldimethylsilyl, triisopropylsilyl, etc. and diphenyl-C₁₋₄ alkylsilyl groups such as diphenylmethylsilyl, diphenylethylsilyl and so on. Still more preferred are tri-C₁₋₄ alkylsilyl groups, etc. and the most preferred is t-butyldimethylsilyl, for instance.

R^{2a} in the above formulas represents an organic group. Since R^{2a} is not involved in the desilylation reaction in the process of this aspect of the invention, it can be virtually any known organic group that is used as the substituent group in the 4-position of azetidinone compounds. To be specific, there can be employed groups of the formula:

-X-CO-W-R²'

wherein X represents S, O or 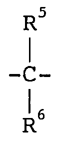
(R⁵ and R⁶ each means hydrogen, C₁₋₄ alkyl or halo-C₁₋₄ alkyl); W represents a bond, N, O or S; R²' represents a hydrocarbon group which may be substituted or a heterocyclic group which may be substituted, by way of example.

The C₁₋₄ alkyl group R⁵ or R⁶ may for example be methyl, ethyl or the like.

The halo-C₁₋₄ alkyl group R⁵ or R⁶ may for example be CH₂F, CF₃ or the like.

The hydrocarbon moiety of said hydrocarbon group which may be substituted for R²', includes (1) saturated or unsaturated C₁₋₆ aliphatic hydrocarbon groups, (2) C₃₋₈ alicyclic hydrocarbon groups and (3) C₆₋ ₁₄ aromatic hydrocarbon groups. Preferred are C₁₋₆ alkyl, C₂₋₆ alkenyl, C₂₋₆ alkynyl, C₃₋₈ cycloalkyl, C₆₋₁₄ aryl and other groups. Still more preferred are C₁₋₆ alkyl, C₆₋₁₄ aryl groups, etc.

The C₁₋₆ alkyl group includes, among others, methyl, ethyl, propyl, isopropyl, butyl, isobutyl, sec-butyl, tert-butyl, etc. Preferred are C₁₋₄ alkyl groups such as methyl, ethyl, propyl, isopropyl and so on.

The C₂₋₆ alkenyl group includes, among others, ethenyl, propenyl, isopropenyl, butenyl, isobutenyl, sec-butenyl, etc. Preferred are C₂₋₄ alkenyl groups such as ethenyl, propenyl, isopropenyl and so on.

The C₂₋₆ alkynyl group includes, among others, ethynyl, propynyl, isopropynyl, butynyl, isobutynyl, sec-butynyl, etc. Preferred are C₂₋₄ alkynyl groups such as ethynyl, propynyl, isopropynyl, etc.

The C₃₋₈ cycloalkyl group includes, among others, cyclopropyl, cyclobutyl, cyclopentyl, cyclohexyl, etc. Preferred are C₃₋₆ cycloalkyl groups such as cyclopropyl, cyclobutyl, etc.

The C₆₋₁₄ aryl group includes, among others, phenyl, naphthyl, anthryl, phenanthryl, etc. Preferred are C₆₋₁₀ aryl groups such as phenyl, naphthyl, etc.

The heterocyclic moiety of said heterocyclic group which may be substituted for R²', includes, among others, 5- or 6-membered heterocyclic groups which may have 1 to 3 nitrogen, sulfur and/or oxygen atoms in addition to carbon atoms and is preferably a 5-membered heterocyclic group containing one nitrogen, sulfur or oxygen atom in addition to carbon atoms, such as pyrrole, pyrroline, thienyl, furyl, etc.; a 6-membered nitrogen-containing group, e.g. a 6-membered heterocyclic group containing 1 or 2 nitrogen atoms in addition to carbon atoms, such as pyridyl, pyrimidyl, pyridazinyl, etc.; a 5-membered nitrogen-containing group, e.g. a 5-membered heterocyclic group containing 2 nitrogen atoms in addition to carbon atoms in addition to carbon atoms, such as pyrazolyl, imidazolyl, etc., a 5-membered heterocyclic group containing 1 nitrogen atom and 1 sulfur atom in addition to carbon atoms, such as thiazolyl etc., a 5-membered heterocyclic group containing 2 nitrogen atoms and 1 sulfur atom in addition to carbon atoms, such as 1,2,3-thiadiazolyl, 1,2,4-thiadiazolyl, 1,3,4-thiadiazolyl, 1,2,5-thiadiazolyl, etc., a 5-membered heterocyclic group containing 2 nitrogen atoms and 1 oxygen atom in addition to carbon atoms, such as 1,2,3-oxadiazolyl, 1,2,4-oxadiazolyl, 1,3,4-oxadiazolyl, 1,2,5-oxadiazolyl, etc., a 5-membered heterocyclic group containing 3 nitrogen atoms in addition to carbon atoms, such as 1,2,5-triazolyl, 1,2,4-triazolyl, etc., and a 5-membered heterocyclic group containing 4 nitrogen atoms and a carbon atom, such as 1H-tetrazolyl, 2H-tetrazolyl, etc. Still more preferred are 5-membered heterocyclic groups containing 1 nitrogen, sulfur or oxygen atom in addition to carbon atoms and 6-membered heterocyclic groups containing 1 or 2 nitrogen atoms in addition to carbon atoms. The most preferred are thienyl, furyl, pyridyl and so on.

The substituent group for said hydrocarbon group which may be substituted or heterocyclic group which may be substituted for R²' includes, among others, (1) hydroxy, (2) oxo, (3) mercapto, (4) halogen, (5) carboxy, (6) carbamoyl, (7) carbamoyloxy, (8) alkoxy, (9) alkoxycarbonyl, (10) alkylthio, (11) alkanoyl, (12) alkanoyloxy, (13) amino optionally mono-substituted or di-substituted by the same or different alkyl or alkanoyl groups, (14) cycloalkyl, (15) aryl, and (16) heterocyclic groups and, where the hydrocarbon group is an alicyclic hydrocarbon group or an aromatic hydrocarbon group, further includes (17) alkyl optionally either mono-substituted or di-substituted by the same or different members selected from among hydroxy, halogen, amino and carboxy. The number of the substituent is one to five, preferably one or two in the possible position(s).

The halogen of (4) includes chlorine, fluorine, bromine and iodine and is preferably chlorine or fluorine.

The alkoxy group of (8) includes, among others, C₁₋₆ alkoxy groups such as methoxy, ethoxy, propoxy, isopropoxy, butoxy, isobutoxy, sec-butoxy, tert-butoxy, etc. Preferred are C₁₋₄ alkoxy groups such as methoxy, ethoxy, propoxy, isopropoxy, etc.

The alkoxy moiety of said alkoxycarbonyl group of (9) includes the same alkoxy groups as mentioned above.

The alkyl moiety of said alkylthio group of (10) includes the same alkyl groups as mentioned above for the hydrocarbon group for R²'.

The alkanoyl group of (11) includes C₁₋₆ alkanoyl groups such as acetoxy, pivaloyloxy, etc. Preferred are C₁₋₄ alkanoyl groups such as acetoxy, etc.

The alkanoyl moiety of said alkanoyloxy group of (12) includes the same alkanoyl groups as mentioned above.

The alkyl or alkanoyl moiety of said amino group which may be mono-substituted or di-substituted by the same or different alkyl or alkanoyl groups of (13) include the same groups as respectively mentioned above. Examples of the optionally substituted amino group include C₁₋₆ alkylamino, di-C₁₋₆ alkylamino and C₁₋₆ alkanoylamino groups such as methylamino, ethylamino, dimethylamino, diethyamino, methylethylamino, acetoxyamino, etc. Preferred are C₁₋₄ alkylamino, di-C₁₋ ₄ alkylamino and C₁₋₄ alkanoylamino groups such as methylamino, ethylamino, dimethylamino, diethylamino, acetoxyamino and so on.

Referring to cycloalkyl of (14), aryl of (15) and heterocyclic groups of (16), the same groups as mentioned for the hydrocarbon group and heterocyclic group for R²', can be employed.

The halogen for said alkyl group which may be mono-substituted or di-substituted by the same or different members of hydroxy, halogen, amino and carboxy of (17) includes the same halogen species as mentioned above. The alkyl group includes the same groups as mentioned above for the hydrocarbon group of R²'. Examples of the optionally substituted alkyl group include, as preferred species, methyl, ethyl, hydroxymethyl, hydroxyethyl, hydroxypropyl, chloromethyl, chloroethyl, fluoromethyl, fluoroethyl, aminomethyl, aminoethyl, carboxymethyl, carboxyethyl, etc. Still more preferred are methyl, ethyl, hydroxymethyl, fluoromethyl, aminomethyl, carboxymethyl and so on.

The preferred substituent group for said hydrocarbon group which may be substituted or said heterocyclic group which may be substituted includes, among others, (a) hydroxy, (b) oxo, (c) halogen, (d) carboxy, (e) carbamoyl, (f) carbamoyloxy, (g) C₁₋₄ alkoxy, (h) amino, (i) C₁₋₆ alkyl groups which may be mono-substituted or di-substituted by the same or different members of hydroxy, halogen, amino and carboxy, etc. Still more preferred are (a) hydroxy, (b) oxo, (e) carbamoyl, (f) carbamoyloxy, (g) C₁₋₄ alkoxy, (h) amino, (i) C₁₋₄ alkyl groups, etc.

Preferred examples of W-R²' include cases in which (i) W represents a bond and R²' represents a C₁₋₆ alkyl group which may be substituted by a carbamoyl, carbamoyloxy or C₁₋₄ alkoxy, (ii) W represents a bond and R²' represents an C₆₋₁₀ aryl group which may be substituted by a carbamoyl, carbamoyloxy or C₁₋₄ alkoxy, (iii) W represents N, O or S and R²' represents a nitrogen, sulfur or oxygen-containing 5-membered heterocyclic group which may be mono- or di-substituted by an oxo, carbamoyl, amino or C₁₋₄ alkyl, (iv) W represents a bond and R²' represents a 6-membered heterocyclic group containing 1 or 2 nitrogen atoms and so on. Still more preferred for R²' are C₁₋₆ alkyl groups which may be substituted by a carbamoyloxy or C₁₋₄ alkoxy, C₆₋₁₄ aryl groups which may be substituted by a carbamoyl, N, S or O-containing 5-membered heterocyclic groups which may be bonding through S and substituted by an oxo, 6-membered heterocyclic groups containing 1 or 2 nitrogen atoms, etc. The most preferred for R²' are C₁₋₄ alkoxy-C₁₋₄ alkyl, carbamoyloxy-C₁₋₄ alkyl, carbamoyl-C₆₋₁₀ aryl, thienylthio, S-oxido-thienylthio, furyl, pyridyl, etc.

The symbol -CO-R^{2b} is an acyl group, and examples of R^{2b} include the groups as mentioned above for R²' and so on.

The preferred example of X is S.

R^{3b} represents a hydrogen atom or a carboxy-protecting group.

The carboxy-protecting group includes, among others, C₁₋₈ alkyl groups such as methyl, ethyl, propyl, isopropyl, butyl, iso-butyl, sec-butyl, tert-butyl, hexyl, etc., C₁₋₆ alkyl groups mono- through tri-substituted by halogen (e.g. chlorine, bromine, fluorine, iodine), such as bromo-tert-butyl, trichloroethyl, etc., C₂₋₆ alkenyl groups such as vinyl, allyl, etc., C₇₋₁₄ aralkyl groups which may be mono- or di-substituted by nitro, C₁₋₄ alkoxy (e.g. methoxy, ethoxy, etc.), C₁₋₄ alkyl (e.g. methyl, ethyl, propyl, iso-propyl, butyl, iso-butyl, sec-butyl, tert-butyl, etc.), etc., such as benzyl, p-nitrobenzyl, o-nitrobenzyl, p-methoxybenzyl, p-tert-butylbenzyl, etc., C₁₋₄ alkanoyloxy-C₁₋₄ alkyl groups such as acetoxymethyl, propionyloxymethyl, butyryloxymethyl, iso-butyryloxymethyl, valeryloxymethyl, pivaloyloxymethyl, 1-(or 2-)-acetoxyethyl, 1-(or 2- or 3-) acetoxypropyl, 1-(or 2-, 3- or 4-)acetoxybutyl, 1-(or 2-)propionyloxyethyl, 1-(or 2- or 3-)propionyloxypropyl, 1-(or 2-)-butyryloxyethyl, 1-(or 2-)isobutyryloxyethyl, 1-(or 2-)pivaloyloxyethyl, 1-(or 2-)hexanoyloxyethyl, isobutyryloxymethyl, 2-ethylbutyryloxymethyl, 3,3-dimethylbutyryloxymethyl, 1-(or 2-)pentanoyloxyethyl, etc., C₁₋₄ alkanesulfonyl-C₁₋₄ alkyl groups such as 2-mesylethyl, etc., C₁₋₄ alkoxycarbonyloxy-C₁₋₄ alkyl groups such as methoxycarbonyloxymethyl, ethoxycarbonyloxymethyl, propoxycarbonyloxymethyl, tert-butoxycarbonyloxymethyl, 1-(or 2-) methoxycarbonyloxyethyl, 1-(or 2-)ethoxycarbonyloxyethyl, 1-(or 2-)isopropoxycarbonyloxyethyl, etc., tri-C₁₋₄ alkylsilyl groups such as tert-butyldimethylsilyl, trimethylsilyl, etc., C₂₋₆ alkenyl groups such as allyl, methallyl, etc., C₁₋₄ alkoxymethyl groups such as methoxymethyl, ethoxymethyl, propoxymethyl, isopropoxymethyl, etc., C₁₋₄ alkylthio-C₁₋₄ alkyl groups such as (2-methylthio)ethyl, etc., 3-methyl-2-butenyl, 5-indanyl, 3-phthalidyl and so on. Preferred are C₇₋₁₄ aralkyl groups which may be mono- or di-substituted by C₂₋₆ alkenyl, nitro, C₁₋₄ alkyl or the like. The most preferred examples are allyl, p-nitrobenzyl and the like.

R^{4a} represents an alkyl group. The alkyl group of R^{4a} is a C₁₋₆ alkyl group such as methyl, ethyl, propyl, isopropyl, butyl, isobutyl, sec-butyl, tert-butyl, etc. Preferred are C₁₋₄ alkyl groups such as methyl, ethyl, propyl, isopropyl, etc.

In the process of this aspect of the invention, an acid is permitted to act on compound (II) C. For example, by admixing compound (II) C and acid can compound (I) C or a salt thereof be produced.

The acid for use in this aspect of the present invention includes inorganic acids such as hydrofluoric acid, hydrochloric acid, etc. and organic acids such as formic acid, acetic acid, etc. Preferred are inorganic acids and the most preferred is hydrochloric acid and the like.

In the process of this aspect of the invention, the ratio of said acid to compound (II) C is about 0.1 ∼ 20 moles and preferably about 1 ∼ 15 moles of the acid per mole of compound (II) C.

In the process of this aspect of the invention, the reaction temperature is not higher than about 30°C and preferably in the range of -10°C ∼ 20°C. The reaction time is about 5 minutes ∼ 24 hours and preferably about 15 minutes ∼ 10 hours.

An organic solvent can be used in the process of this aspect of the invention. The organic solvent is not critical in type only if it does not interfere with the reaction. Thus, for example, ethyl acetate, tetrahydrofuran, acetonitrile, acetone, etc. can be employed. The amount of the solvent is 1 ∼ 30 times and preferably 1 ∼ 20 times volume to the compound (II) C.

Where the resultant compound (I) C is a free acid, it may be converted to a pharmacologically acceptable salt in the conventional manner. The salt of compound (I) C includes alkali metal salts such as sodium salt, potassium salt and so on. Where compound (I) C has a basic group, it can be converted to a salt with e.g. an inorganic acid, such as hydrochloride, in the conventional manner.

Compound (I) C or a salt thereof can be separated and purified by the known isolation procedures such as concentration, extraction, washing, chromatography, crystallization, recrystallization, etc. However, omitting the isolation and purification step, the reaction mixture as it is can be used as a starting material for the synthesis of penem compounds.

The starting compound (II) C including the known compound can be synthesized by the processes described or implied in inter alia EP-A-199675, U. S. Patent 4,826,832, Chemical Pharmaceutical Bulletin 31 (2), 768-771 (1983) and Tetrahedron Letters 25, No. 22, 2395-2398 (1984).

The compounds of formula (I') C, wherein
R^{1c} is a hydrogen atom are subsumed in the category of compound (I) C which can be obtained by the process of this aspect of the invention, and salts thereof are novel compounds. The starting compound (I') C wherein R^{1c} is a silyl group, of the formula: 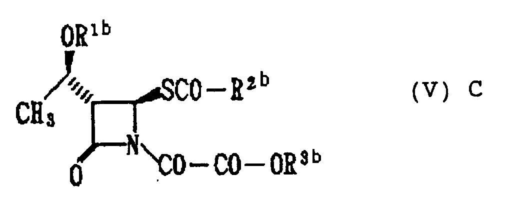
wherein the symbols have the same meanings as defined hereinbefore, can be synthesized from a compound of the formula: 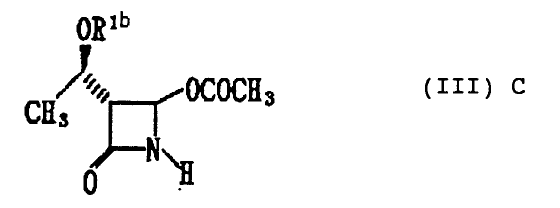
wherein the symbols have the same meanings as defined hereinbefore, which is an intermediate of penem antibiotics, for example by the process shown below by way of reaction schema. 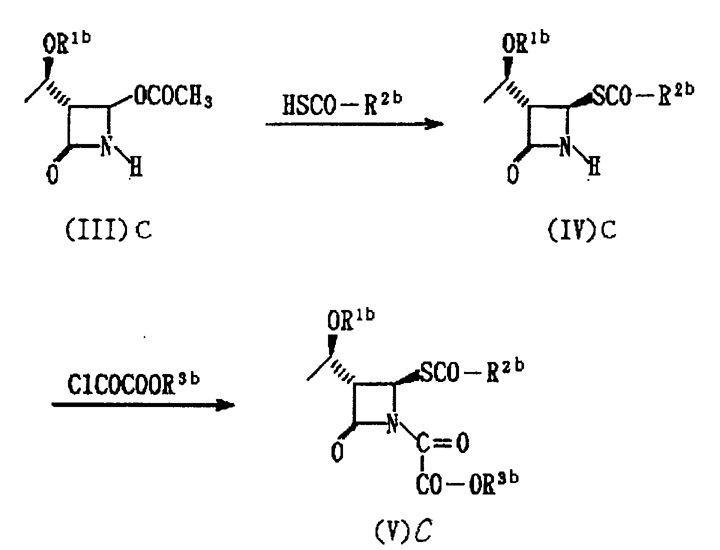
wherein the symbols have the same meanings as defined hereinbefore.

Thus, compound (III) C is reacted with a compound of the formula HSCO-R^{2b} wherein the symbol has the same meaning as defined hereinbefore, to replace the acetoxy group with a group of the formula SCO-R^{2b} wherein the symbol has the same meaning as defined hereinbefore to thereby provide compound (IV) C. This reaction is carried out in a binary solvent system comprising an inert organic solvent (dichloromethane, ethyl acetate, toluene, etc.) and water, preferably dichloromethane-H₂O. The compound HSCO-R^{2b} is used in a proportion of about 1 ∼ 3 moles, preferably about 1 ∼ 1.5 moles per mole of compound (III) C. The reaction temperature is about 0°C ∼ 40°C and preferably about 10°C ∼ 30°C. The reaction time is about 0.5 hour ∼ 30 hours and preferably about 1 hour ∼ 24 hours.

Compound (IV) C is reacted with a compound of the formula CℓCOCOOR^{3b} wherein the symbol has the same meaning as defined hereinbefore, in the presence of a tri(lower)alkylamine such as triethylamine, N,N-diisopropylethylamine, etc., preferably triethylamine or N,N-diisopropylethylamine, to provide a compound (V) C having a group of the formula COCOOR^{3b} wherein the symbol has the same meaning as defined hereinbefore. The compound of the formula CℓCOCOOR^{3b} is used in a proportion of about 1 ∼ 2 moles, preferably about 1 ∼ 1.5 moles, per mole of compound (IV) C. This reaction is conducted in an inert organic solvent (dichloromethane, ethyl acetate, toluene, etc.), preferably dichloromethane. The reaction temperature is about -40°C ∼ 30°C and preferably about -20°C ∼ 20°C. The reaction time is not more than about 2 hours and preferably about 3 ∼ 90 minutes.

The resultant compound (V) C can be subjected to desilylation reaction in accordance with this aspect of the invention and, then, conversion to a penem ester compound having excellent antibacterial activity as shown below by reaction schema. 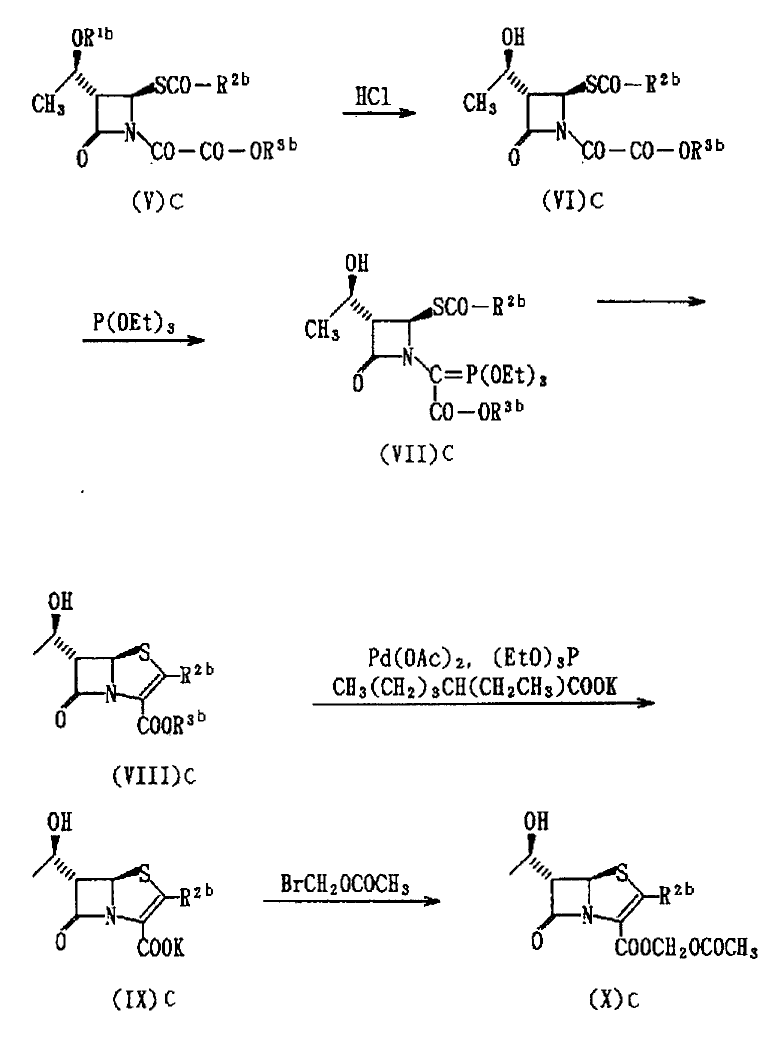
wherein the symbols have the same meanings as defined hereinbefore.

Thus, by the process of this aspect of the invention, compound (V) C can be reacted with hydrochloric acid to remove the silyl group and thereby provide hydroxyethylazetidinone (VI) C. Hydrochloric acid is used in a proportion of about 1 ∼ 20 moles, preferably about 2 ∼ 15 moles, per mole of compound (V) C. The solvent which can be used for this reaction is not critical only if it does not interfere with the reaction. Preferably, the reaction is carried out in acetonitrile. The reaction temperature is not higher than about 25°C and is preferably between -10°C and 15°C. The reaction time is about 5 minutes ∼ 24 hours and preferably about 15 minutes ∼ 5 hours. The resultant compound (VI) C can be separated and purified by the known isolation procedures mentioned hereinabove.

The resultant compound (VI) C can be reacted with triethyl phosphite to give compound (VII) C. This reaction can be conducted in the absence of a solvent or in an inert organic solvent (e.g. dichloromethane, xylene, toluene, dioxane, etc.). Triethyl phosphite is used in a proportion of generally about 2 ∼ 50 moles and preferably about 5 ∼ 40 moles per mole of compound (VI) C. The reaction temperature is about 20°C ∼ 80°C and preferably about 30°C ∼ 70°C. The reaction time is about 0.5 ∼ 10 hours and preferably about 1 ∼ 4 hours.

Compound (VII) C is heated in 2-methyl-1-propyl alcohol to provide compound (VIII) C. The concentration of compound (VII) C in the alcohol is about 1 ∼ 50% (w/w) and preferably about 5 ∼ 30% (w/w). The heating temperature is about 30 ∼ 100°C and preferably about 50°C ∼ 90°C. The reaction time is about 30 minutes ∼ 15 hours and preferably about 1 ∼ 10 hours.

Then, compound (VIII) C is subjected to the removing reaction of the carboxy-protecting group in the conventional manner, or reacted with potassium 2-ethylhexanoate in the presence of palladium acetate and triethyl phosphite to remove the carboxy-protecting allyl group and thereby provide compound (IX) C. Potassium 2-ethylhexanoate is used in a proportion of about 1 ∼ 4 moles, preferably about 1 ∼ 2 moles, per mole of compound (VIII) C. Palladium acetate is used in a catalyst amount, namely about 0.0001 ∼ 0.1 mole, preferably about 0.001 ∼ 0.05 mole, per mole of compound (VIII) C. Triethyl phosphite is used in a proportion of about 2 ∼ 20 moles, preferably about 3 ∼ 7 moles, per mole of palladium acetate. This reaction is conducted in the presence of a solvent. There is no particular limitation on the type of solvent insofar as it does not interfere with the reaction, but the reaction can, for example, be carried out in an inert organic solvent such as THF (tetrahydrofuran), methylene chloride or a mixture thereof, preferably THF-methylene chloride. The reaction temperature is about -20°C ∼ 50°C and preferably about 0°C ∼ 40°C. The reaction time is about 5 minutes ∼ 8 hours and preferably about 15 minutes ∼ 5 hours.

Compound (IX) C can be reacted with, for example, bromomethyl acetate to provide acetoxymethyl ester (X) C. Bromomethyl acetate is used in a proportion of about 1 ∼ 4 moles, preferably about 1 ∼ 1.5 moles, per mole of compound (IX) C. This reaction is carried out in an inert organic solvent such as dimethylformamide, dimethylacetamide, acetonitrile, etc., preferably dimethylacetamide or the line. The reaction temperature is about -30°C ∼ 50°C and preferably about -20°C ∼ 35°C. The reaction time is about 10 minutes ∼ 5 hours and preferably about 30 minutes ∼ 3 hours.

In the above processes from the compound (III) C to the compound (X) C, each of the produced compounds can be separated and purified by the known isolation procedures such as concentration, extraction, washing, chromatography, crystallization, recrystallization and so on, but can be used in the reaction mixture without isolation as the starting material for the next step.

The penem ester compounds[e.g. (X) C] synthesized by using a synthetic intermediate (I) C, inclusive of their pharmacologically acceptable salts, have excellent antibacterial activity and are of great value as safe antibacterial drugs for the therapy and prophylaxis of bacterial infections associated with a broad spectrum of pathogenic bacteria. In using such compound as an antibacterial agent, it can be safely administered in the form of a pharmaceutical composition (such as tablets, granules, injections, etc.) containing its antibacterially effective amount to man and other mammals (e.g. mouse, dog, cat, etc.). For oral administration, the compound is formulated and processed into tablets, capsules, etc. by the established pharmaceutical procedures. Such dosage forms may contain a variety of additives, e.g. diluents such as lactose, glucose, sucrose, mannitol, sorbitol, cellulose, glycine, etc., lubricants such as silica, talc, stearic acid or its salt, polyethylene glycol, etc., binders such as magnesium aluminosilicate, starch, gelatin, gums, cellulose derivatives, polyvinylpyrrolidone, etc., disintegrators such as starch, alginic acid or its salt, etc., colorants, perfumes, sweeteners and so on. The dosage can be varied over a broad range according to the patient's age, body weight and clinical condition, dosage form, doctor's diagnosis, etc. but generally speaking the dosage range of about 50 ∼ 1,000 mg per day can be used as the standard regimen for adult patients (body weight 50 kg). Usually, this dosage can be administered orally, parenterally or locally in a single dose or in a few divided doses.

The following Test Example, Reference Examples and Examples are intended to describe the invention in further detail and should by no means be construed as defining the metes and bounds of the invention.

The NMR spectra given in the following examples were determined with Hitachi R-90H (90 MHz) spectrometer using tetramethylsilane as the internal standard. All chemical shifts (δ values) are shown in ppm. The symbols used have the following meanings.
- s:: singlet
- d:: doublet
- t:: triplet
- ABq:: AB quartet
- dd:: double doublet
- dq:: double quartet
- dt:: double triplet
- m:: multiplet
- br.:: broad
- J:: coupling constant
- sh.:: shoulder
- Et:: ethyl
- Ph:: phenyl
- %:: % by weight, unless otherwise indicated
- Room temperature:: 20-25°C

### Example 1

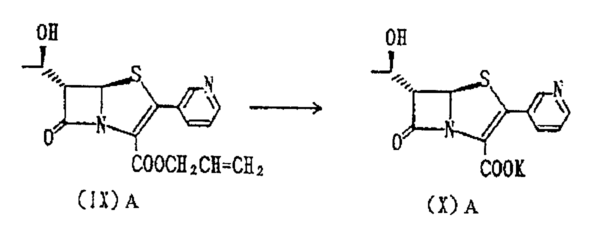

In a mixed solvent comprising 13 ml of THF and 25 ml of methylene chloride was dissolved 3.41 g (content 97.6 %) of allyl (5R,6S)-6-[(R)-1-hydroxyethyl]-7-oxo-3-(3-pyridyl)-4-thia-1-azabicyclo[3.2.0]hept-2-ene-2- carboxylate, followed by addition of 87.5 g of triethyl phosphite and 22.5 g of palladium acetate. A solution of potassium 2-ethylhexanoate prepared by mixing a THF solution (12 ml) of 0.67 g potassium hydroxide with 1.73 g of 2-ethylhexanoic acid under stirring at room temperature for 30 minutes was added to the above solution and the mixture was stirred at 30°C for 2.5 hours.

The precipitated potassium (5R,6S)-6-[(R)-1-hydroxyethyl]-7-oxo-3-(3-pyridyl)-4-thia-1-azabicyclo[3.2.0]hept-2-ene-2-carboxylate (I) was recovered by filtration, washed with 30 ml of methylene chloride and dried in vacuo to provide 3.43 g of crystals (content 82.8 %; yield 86.0%), (water content 3.4 %).
NMR (DMSO-d₆): δ 1.17 (3H, d, J=6 Hz), 3.6' (1H, dd, J=1.5 x 6 Hz), 3.95 (1H, dq, J=6 x 6 Hz), 5.59 (1H, d, J=1.5 Hz), 7.2-8.7 (4H, m) ppm.

### Example 2

In a mixed solvent comprising 13 ml of THF and 25 ml of methylene chloride was dissolved 3.41 g (content 97.6 %) of allyl (5R,6S)-6-[(R)-1-hydroxyethyl]-7-oxo-3-(3-pyridyl)-4-thia-1-azabicyclo[3.2.0]hept-2-ene-2-carboxylate, followed by addition of 170.1 mg of triethyl phosphite and 45.0 mg of palladium acetate. A solution of potassium 2-ethylhexanoate prepared by mixing a THF solution (12 ml) of 0.65 g potassium hydroxide with 1.95 g of 2-ethylhexanoic acid under stirring at room temperature for 30 minutes was added to the above solution and the mixture was stirred at 30°C for 1.5 hours.

The precipitated potassium (5R,6S)-6-[(R)-1-hydroxyethyl]-7-oxo-3-(3-pyridyl)-4-thia-1-azabicyclo[3.2.0]hept-2-ene-2-carboxylate (I) was recovered by filtration, washed with 30 ml of methylene chloride and dried in vacuo to provide 3.57 g of crystals (content 83.2 %; yield 89.9%), (water content 2.7 %).

### Example 3

In a mixed solvent comprising 10 ml of THF and 20 ml of methylene chloride was dissolved 3.37 g (content 98.6 %) allyl (5R,6S)-6-[(R)-1-hydroxyethyl]-7-oxo-3-(3-pyridyl)-4-thia-1-azabicyclo[3,2,0]hept-2-ene-2-carboxylate, followed by addition of 199.4 mg of triethyl phosphite and 90.0 mg of palladium acetate. A solution of potassium 2-ethylhexanoate prepared by mixing a THF solution (10 ml) of 0.68 g potassium hydroxide with 1.95 g of 2-ethylhexanoic acid and 360 mg of water under stirring at room temperature for 30 minutes was added to the above solution and the mixture was stirred at 35°C for 10 minutes.

The precipitated potassium (5R,6S)-6-[(R)-1-hydroxyethyl]-7-oxo-3-(3-pyridyl)-4-thia-1-azabicyclo[3,2,0]hept-2-ene-2-carboxylate (I) was recovered by filtration, washed with 100 ml of methylene chloride and dried in vacuo to provide 3.53 g of crystals (content 91.8 %; yield 98.1 %), (water content 6.8 %).

### Test Example

Allyl (3S,4R)-3-[(R)-1-(tert-butyldimethylsilyloxy)ethyl]-4-nicotinoylthio-2-oxo-α-triethoxyphosphoranedioyl-1-azetidine acetate [hereinafter referred to briefly as (A)] was dissolved in 10 volumes each of various solvents. Each solution was stirred at 50°C for 2 hours, at the end of which time it was analyzed by high performance liquid chromatography to determine the percentage yield of allyl (5R,6S)-6-[(R)-1-(tert-butyldimethylsilyloxy)ethyl]-2-(3-pyridyl)-2-penem-3-carboxylate [hereinafter referred to as (B)] and the percentage residue of (A). The data corresponding to non-alcoholic solvents are presented in Table 1 and those corresponding to alcoholic solvents are presented in Table 2.

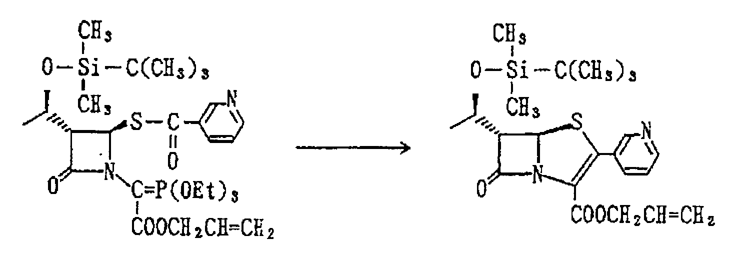

**Table 1**

| Solvent | % Residue of (A) | % Yield of (B) |
|---|---|---|
| Xylene | 102.4 | 1.1 |
| Hexane | 107.1 | 1.2 |
| Carbon tetrachloride | 104.5 | 1.6 |
| Nitromethane | 94.6 | 4.5 |
| 1,2-Dichloroethane | 99.5 | 4.2 |
| Diphenylmethane | 98.3 | 1.1 |
| Isopropyl ether | 101.9 | 1.0 |
| Dioxane | 99.5 | 1.1 |
| Tetrahydrofuran | 102.7 | 2.7 |
| Acetonitrile | 99.1 | 3.5 |
| Propionitrile | 98.0 | 3.9 |
| Acrylonitrile | 99.3 | 4.8 |
| Acetone | 99.3 | 1.8 |
| Methyl ethyl ketone | 97.9 | 2.9 |
| 2,4-Pentadione | 96.4 | 3.4 |
| Ethyl acetate | 99.4 | 1.2 |
| Diethyl malonate | 95.1 | 2.2 |
| N,N-Dimethylformamide | 95.3 | 3.3 |
| N,N-Dimethylacetamide | 95.5 | 3.7 |
| Dimethyl sulfoxide | 91.4 | 4.9 |
| Acetic acid | 0.1 | 2.5 |

**Table 2**

| Solvent | % Residue of (A) | % Yield of (B) |
|---|---|---|
| Methyl alcohol | 56.6 | 41.0 |
| Ethyl alcohol | 68.7 | 29.0 |
| 1-Propyl alcohol | 66.1 | 29.9 |
| 1-Butyl alcohol | 69.6 | 27.5 |
| 2-Methyl-1-propyl alcohol | 69.4 | 28.9 |
| 1-Pentyl alcohol | 80.9 | 30.1 |
| 1-Octyl alcohol | 72.0 | 26.3 |
| Ethyl glycolate | 65.8 | 23.7 |
| 2-Propyl alcohol | 81.1 | 19.1 |
| 2-Butyl alcohol | 82.2 | 15.2 |
| 2-Pentyl alcohol | 83.0 | 17.2 |
| 3-Pentyl alcohol | 81.5 | 16.9 |
| Cyclopentyl alcohol | 81.7 | 18.2 |
| Cyclohexyl alcohol | 72.0 | 19.4 |
| Allyl alcohol | 62.4 | 34.4 |
| Benzyl alcohol | 62.5 | 38.9 |
| 2,2,2-Trichloroethyl alcohol | 30.9 | 59.0 |
| 2-Methoxyethyl alcohol | 79.3 | 17.7 |
| 2,2,2-Trifluoroethyl alcohol | 5.8 | 50.8 |

The above test revealed that the process of the invention which employs an alcoholic solvent provides for the production of compound (B) in a remarkably increased amount (yield) at a lower temperature (50°C) in a reduced reaction time (2 hours) with an accelerated rate (higher reaction velocity) as compared with processes employing other solvents.

### Reference Example 1

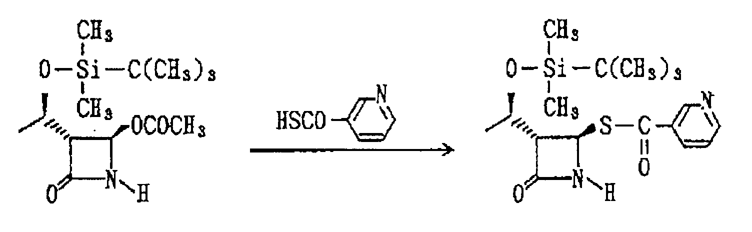

In 230 ml of dichloromethane was dissolved 28.7 g (0.1 mole) of (3R,4R)-4-acetoxy-3-[(R)-1-(tert-butyldimethylsilyloxy)ethyl]-2-azetidinone and the solution was added to a solution of 5.6 g sodium hydroxide and 18.2 g of thionicotinic acid in 180 ml of water. The mixture was stirred at 25°C for 24 hours. After phase separation, the water layer was extracted with 73 ml of dichloromethane and the organic layers were combined. This organic solution was washed serially with 100 ml of saturated NaHCO₃-H₂O and 100 ml of saturated NaCl-H₂O, dried over anhydrous magnesium sulfate and concentrated to dryness under reduced pressure. The residue was suspended in 150 ml of n-hexane and the suspension was stirred and then allowed to sand in the cold. The resultant crystals were collected by filtration, washed with 50 ml of n-hexane and dried under reduced pressure to provide 32.6 g (yield 88.9%) of (3S,4R)-3-[(R)-1-(tert-butyldimethylsilyloxy)ethyl]-4-nicotinoylthio-2-azetidinone.
NMR (CDCl₃): δ 0.10 (6H, s), 0.90 (9H, s), 1.26 (3H, d, J = 6.2 Hz), 3.31 (1H, dd, J = 2.6 x 4 Hz), 4.32 (1H, dq, J = 4 x 6.2 Hz), 5.52 (1H, d, J = 2.6 Hz), 6.40 (1H, br. s), 7.3-9.2 (4H, m) ppm

### Reference Example 2

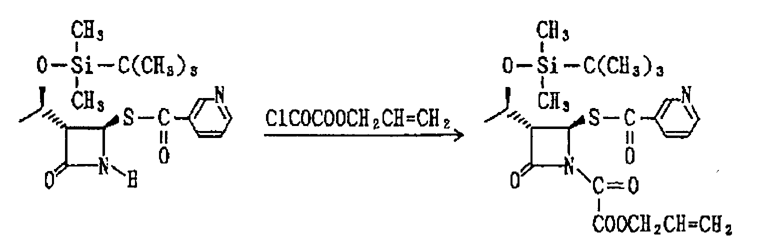

In 536 ml of dichloromethane was dissolved 111.8 g (0.3 mole) of (3S,4R)-3-[(R)-1-(tert-butyldimethylsilyloxy)ethyl]-4-nicotinoylthio-2-azetidinone, followed by dropwise addition of 55.4 g of N,N-diisopropylethylamine with stirring at -5^{∼}-10°C. Then, 57.9 g of allyloxyoxalyl chloride was added dropwise and the mixture was stirred at -5°C for 15 minutes. The reaction mixture was added to 335 ml of iced water and the mixture was stirred for 5 minutes. After phase separation, the water layer was extracted with 120 ml of dichloromethane and the organic layers were combined. This organic solution was washed with 350 ml of water, dried over anhydrous magnesium sulfate and concentrated to dryness under reduced pressure. The residue was dissolved in 120 ml of dichoromethane followed by dropwise addition of 1800 ml of n-hexane and the mixture was stirred at room temperature for 1 hour. The resultant crystals were collected by filtration, washed with 500 ml of n-hexane and dried under reduced pressure to provide 118.3 g (yield 80.1%) of allyl (3S,4R)-3-[(R)-1-(tert-butyldimethylsilyloxy)ethyl]-4-nicotinoyl-2-oxo-1-azetidine glyoxylate.
NMR (CDCl₃): δ 0.07 (3H, s), 0.12 (3H, s), 0.88 (9H, s), 1.29 (3H, d, J = 6.4 Hz), 3.61 (1H, dd, J = 3 x 3 Hz), 4.42 (1H, dq, J = 3 x 6.4 Hz), 4.7-6.2 (5H, m), 6.20 (1H, d, J = 3 Hz), 7.3-9.2 (4H, m) ppm

### Reference Example 3

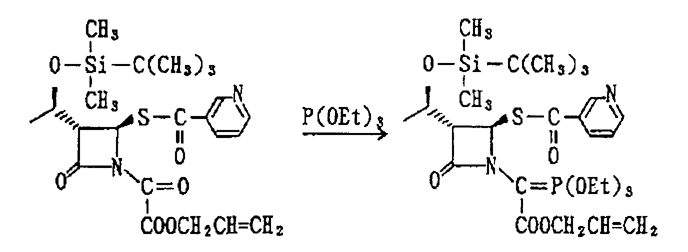

In 99.7 g of triethyl phosphite was dissolved 9.8 g (0.02 mole) of allyl (3S,4R)-3-[(R)-1-(tert-butyldimethylsilyloxy)ethyl]-4-nicotinoylthio-2-oxo-1-azetidine glyoxylate and the solution was stirred at 50°C for 2 hours. The reaction mixture was concentrated under reduced pressure and the resultant oil was dissolved in 170 ml of n-hexane. The solution was washed with water (60 ml H₂O x 2), dried over anhydrous magnesium sulfate and concentrated under reduced pressure to provide 14.0 g (yield 90.3%) of allyl (3S,4R)-3-[(R)-1-(tert-butyldimethylsilyloxy)ethyl]-4-nicotinoylthio-2-oxo-α-triethoxyphosphoranedioyl-1-azetidine acetate.
NMR (CDCl₃) : δ 0.10 (6H, s), 0.84 (9H, s), 1.3 (12H, m), 3.2 (1H, br.s), 4.2 (7H, m), 4.6 (2H, br.s), 5.2 (2H, m), 5.8 (1H, br.s), 5.9 (1H, m), 7.41 (1H, dd), 8.17 (1H, dt), 8.8 (1H, dd), 9.13 (1H, dd) ppm. SIMS: m/e 465 (MH⁺)

### Reference Example 4

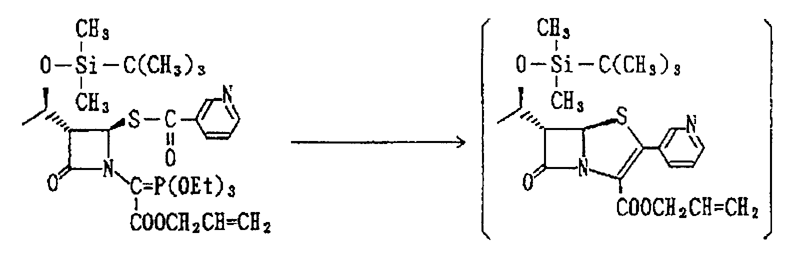

In 5.9 ml of xylene was dissolved 1.011 g (0.920 mmole) of allyl (3S,4R)-3-[(R)-1-(tert-butyldimethylsilyloxy)ethyl]-4-nicotinoylthio-2-oxo-α- triethoxyphosphoranedioyl-1-azetidine acetate and the solution was refluxed for 40 minutes. Analysis of the reaction mixture by high performance liquid chromatography revealed that the yield of allyl (5R,6S)-6-[(R)-1-(tert-butyldimethylsilyloxy)ethyl]-2-(3-pyridyl)-2-penem-3-carboxylate was 68.7%.

### Example 4

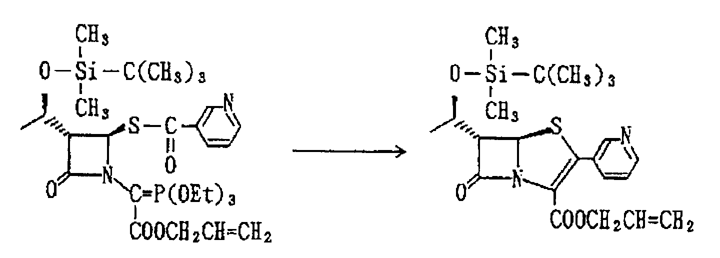

In 100 g of isobutyl alcohol was dissolved 10.0 g of allyl (3S,4R)-3-[(R)-1-(tert-butyldimethylsilyloxy)ethyl]-4-nicotinoylthio-2-oxo-α-triethoxyphosphoranedioyl-1-azetidine acetate and the solution was stirred at 80°C for 2.5 hours. The solvent was then distilled off and the residue was dissolved in n-hexane. The solution was washed with 10% NaCl-H₂O and water in that order and dried over anhydrous magnesium sulfate. The residue was subjected to silica gel chromatography [silica gel 42.4 g, eluent n-hexane-ethyl acetate (7:3)]. The active fractions were pooled and concentrated to dryness under reduced pressure and the residue was allowed to stand in the cold to provide 4.8 g (yield 87%) of allyl (5R,6S)-6-[(R)-1-(tert-butyldimethylsilyloxy)ethyl]-2-(3-pyridyl)-2-penem-3-carboxylate as crystals.
NMR (CDCl₃) : δ 0.10 (6H, s), 0.91 (9H, s), 1.28 (3H, d, J = 6 Hz), 3.80 (1H, dd, J = 1.5 x 4.5 Hz), 4.29 (1H, dq, J = 4.5 x 6 Hz), 4.5-6.1 (5H, m), 5.74 (1H, d, J = 1.5 Hz), 7.2-8.8 (4H, m) ppm

### Example 5

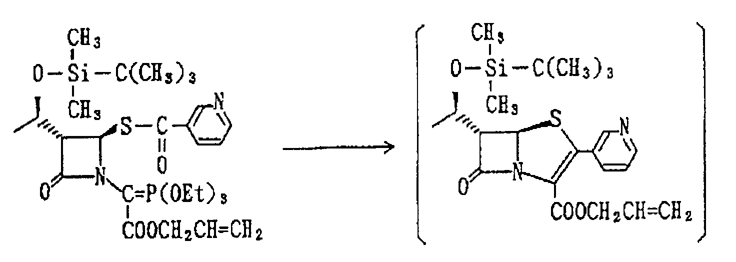

In 10.2 g of isobutyl alcohol was dissolved 1.018 g of allyl (3S,4R)-3-[(R)-1-(tert-butyldimethylsilyloxy)ethyl]-4-nicotinoylthio-2-oxo-α-triethoxyphosphoranedioyl-1-azetidine acetate and the solution was stirred at 80°C for 2.5 hours. Analysis of the reaction mixture by HPLC revealed that the yield of allyl (5R,6S)-6-[(R)-1-(tert-butyldimethylsilyloxy)ethyl]-2-(3-pyridyl)-2-penem-3-carboxylate was 91.2%.

### Example 6

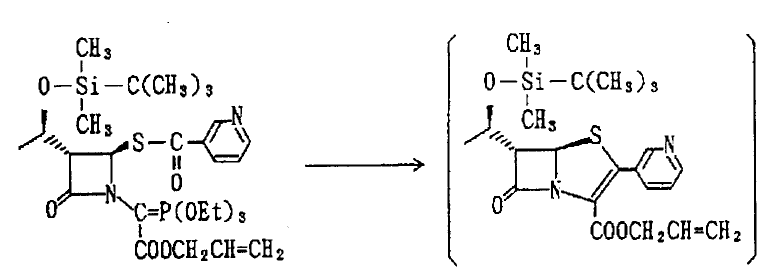

In 15.5 g of benzyl alcohol was dissolved 1.551 g (2.0 mmoles) of allyl (3S,4R)-3-[(R)-1-(tert-butyldimetylsilyloxy)ethyl]-4-nicotinoylthio-2-oxo-α-triethoxyphosphoranedioyl-1-azetidine acetate and the solution was stirred at 80°C for 2.5 hours. Analysis of the reaction mixture by HPLC revealed that the yield of allyl (5R,6S)-6-[(R)-1-(tert-butyldimethylsilyloxy)ethyl]-2-(3-pyridyl)-2-penem-3-carboxylate was 90.5%.

### Example 7

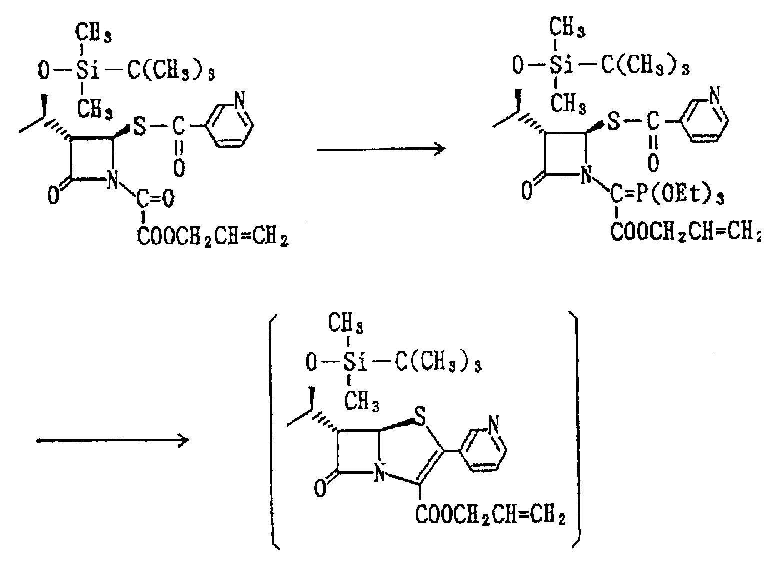

In 10.0 g of triethyl phosphite was dissolved 0.984 g of allyl (3S,4R)-3-[(R)-1-(tert-butyldimethylsilyloxy)ethyl]-4-nicotinoylthio-2-oxo-1-azetidine glyoxylate and the solution was stirred at 50°C for 2 hours. The reaction mixture was then concentrated under reduced pressure followed by addition of 21.4 g of isobutyl alcohol and the mixture was stirred at 80°C for 2.5 hours. Analysis of this reaction mixture by HPLC revealed that the percentage yield of allyl (5R,6S)-6-[(R)-1-(tert-butyldimetylsilyloxy)ethyl]-2-(3-pyridyl)-2-penem-3-carboxylate was 85.0%.

### Example 8

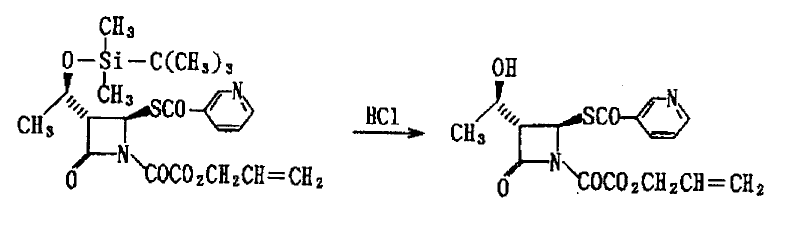

Allyl (3S,4R)-3-[(R)-1-(tert-butyldimethylsilyloxy)ethyl]-4-nicotinoylthio-2-oxo-1-azetidineglyoxylate (10.15 g) was dissolved in CH₃CN (203 ml) and after dropwise addition of concentrated hydrochloric acid (20 ml) under ice-cooling, the solution was stirred at the same temperature for 3 hours.

The reaction mixture was neutralized with a saturated aqueous solution of NaHCO₃ (185 ml) and the organic layer was washed with a saturated aqueous solution of NaCℓ and dried over MgSO₄. The solvent was then distilled off and the residue was washed with hexane (100 ml) to provide 6.77 g (88%) of allyl (3S,4R)-3-[(R)-1-hydroxyethyl]-4-nicotinoylthio-2-oxo-1-azetidineglyoxylate as white crystals.
IR^{KBr} (cm⁻¹): 3240, 1814, 1714, 1680, 908
NMR (CDCl₃) δ: 1.38 (3H, d, J=6.4 Hz), 2.88 (1H, s), 3.65 (1H, dd, J=3.5 Hz & 5.2 Hz), 4.43 (1H, dq, J=5.2 Hz & 6.4 Hz), 4.7 ∼ 6.1 (5H, m), 6.09 (1H, d, J=3.5 Hz), 7.4 ∼ 9.2 (4H, m) ppm.

### Example 9

Allyl (3S,4R)-3-[(R)-1-(tert-butyldimethylsilyloxy)ethyl]-4-nicotinoylthio-2-oxo-1-azetidineglyoxylate (7.52 g) was dissolved in CH₃CN (15 ml) and after dropwise addition of concentrated hydrochloric acid (4.6 ml) under ice-cooling, the solution was stirred at the same temperature for 6 hours.

After CH₂Cl₂ (30 ml) was added, the reaction mixture was neutralized with a saturated aqueous solution of NaHCO₃ (55.5 ml) and the organic layer was washed with water (15 ml). The solvent was then distilled off and the residue was washed with hexane (100 ml) to provide 5.18 g (91%) of allyl (3S,4R)-3-[(R)-1-hydroxyethyl]-4-nicotinoylthio-2-oxo-1-azetidineglyoxylate as white crystals.

### Example 10

Allyl (3S,4R)-3-[(R)-1-(tert-butyldimethylsilyoxy)ethyl]-4-nicotinoylthio-2-oxo-1-azetidineglyoxylate (1.03 g) was dissolved in acetone (10 ml) and after dropwise addition of concentrated hydrochloric acid (2 ml) under ice-cooling, the solution was stirred at the same temperature for 6 hours.

To the reaction mixture was added a saturated aqueous solution of NaHCO₃ to saturation (33 ml) and extracted with AcOEt (15 ml). The organic layer was washed with a saturated aqueous solution of NaCl and dried over MgSO₄. The solvent was then distilled off and the residue was washed with hexane (14 ml) to provide 0.57 g (73%) of allyl (3S,4R)-3-[(R)-1-hydroxyethyl]-4-nicotinoylthio-2-oxo-1-azetidineglyoxylate as white crystals.

### Example 11

Allyl (3S,4R)-3-[(R)-1-(tert-butyldimethylsilyloxy)ethyl]-4-nicotinoylthio-2-oxo-1-azetidineglyoxylate (0.97 g) was dissolved in THF (20 ml) and after dropwise addition of concentrated hydrochloric acid (2 ml) under ice-cooling, the solution was stirred at the same temperature for 18 hours. Analysis of the reaction mixture by high performance liquid chromatography revealed that the yield of allyl (3S,4R)-3-[(R)-1-hydroxyethyl]-4-nicotinoylthio-2-oxo-1-azetidineglyoxylate was 75%.

### Example 12

Allyl (3S,4R)-3-[(R)-1-(tert-butyldimethylsilyloxy)ethyl]-4-nicotinoylthio-2-oxo-1-azetidineglyoxylate (0.97 g) was dissolved in THF (5 ml) and after dropwise addition of concentrated hydrochloric acid (2 ml) under ice-cooling, the solution was stirred at the same temperature for 5 hours. Analysis of the reaction mixture by high performance liquid chromatography revealed that the yield of allyl (3S,4R)-3-[(R)-1-hydroxyethyl]-4-nicotinoylthio-2-oxo-1-azetidineglyoxylate was 90%.

### Example 13

Allyl (3S,4R)-3-[(R)-1-(tert-butyldimethylsilyloxy)ethyl]-4-nicotinoylthio-2-oxo-1-azetidineglyoxylate (0.97 g) was dissolved in AcOEt (20 ml) and after dropwise addition of concentrated hydrochloric acid (2 ml) under ice-cooling, the solution was stirred at the same temperature for 5 hours. Analysis of the reaction mixture by high performance liquid chromatography revealed that the yield of allyl (3S,4R)-3-[(R)-1-hydroxyethyl]-4-nicotinoylthio-2-oxo-1-azetidineglyoxylate was 87%.

### Reference Example 5

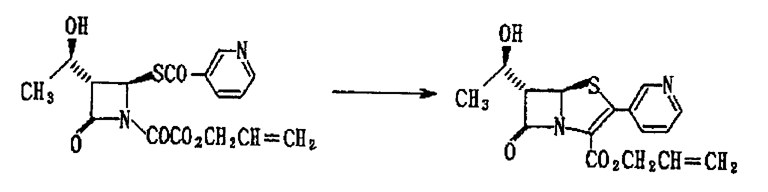

To allyl (3S,4R)-3-[(R)-3-hydroxyethyl]-4-nicotinoylthio-2-oxo-1-azetidineglyoxylate (4.12 g) was added triethyl phosphite (62 ml) and the mixture was stirred at 50°C for 1 hour. This reaction mixture was concentrated and after addition of i-BuOH (219 ml), the mixture was stirred at 80°C for 5 hours.

The reaction mixture was then concentrated, hexane (150 ml) was added to the residue and the mixture was stirred for 30 minutes. The resultant crystals were collected by filtration and washed with hexane (20 ml) and water (17 ml) to provide 3.03 g (81%) of allyl (5R,6S)-6-[(R)-1-hydroxyethyl]-7-oxo-3-(3-pyridyl)-4-thia-1-azabicyclo[3.2.0]hept-2-ene-2-carboxylate.

## Claims

1. A process for producing a compound of the formula: 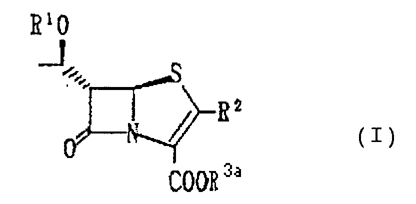 wherein R¹ represents a hydrogen atom or a hydroxy-protecting group, R² represents an organic group and R^{3a} represents a hydrogen atom or an acetoxymethyl group or a salt thereof, which comprises reacting a compound of the formula: 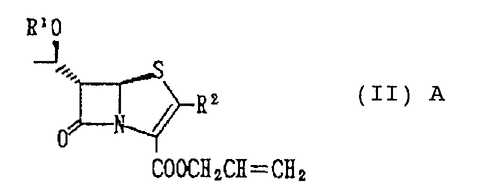 wherein all the symbols have the meanings defined above with an allyl acceptor in the presence of a divalent palladium salt and a reducing agent for said salt to produce a compound (I) wherein R^{3a} represents a hydrogen atom or a salt thereof, and in the case of producing a compound (I) wherein R^{3a} represents an acetoxymethyl group, further reacting thus obtained compound (I) wherein R^{3a} represents a hydrogen atom or a salt thereof with a halogenomethyl acetate, and if necessary removing the protecting group.

2. A process according to claim 1, wherein the divalent palladium salt is palladium acetate or palladium chloride.

3. A process according to claim 1, wherein the reaction of the compound (II) A and the allyl acceptor is carried out in a solvent comprising tetrahydrofuran and methylene chloride.

4. A process according to claim 1, wherein the allyl acceptor is an alkali metal 2-alkylhexanoate.

5. A process according to claim 1, wherein the allyl acceptor is potassium 2-ethylhexanoate.

6. A process according to claim 1, wherein R¹ represents a hydrogen atom.

7. A process according to claim 1, wherein R² represents a pyridyl group.

8. A process according to claim 1, wherein the organic group is a group of the formula:
-W-R²'
wherein W represents a bond, N, O or S and R²' represents a substituted or unsubstituted (1) C₁₋₆ alkyl, (2) C₂₋₆ alkenyl, (3) C₂₋₆ alkynyl, (4) C₃₋₈ cycloalkyl, (5) C₆₋₁₀ aryl or (6) 5- or 6-membered heterocyclic group containing 1 to 3 nitrogen, sulfur and/or oxygen atom(s), the substituent(s) being one to five selected from the group consisting of a (i) hydroxyl, (ii) oxo, (iii) mercapto, (iv) halogen, (v) carboxyl, (vi) carbamoyl, (vii) carbamoyloxy, (viii) C₁₋₆ alkoxy, (ix) C₁₋₆ alkoxy-carbonyl, (x) C₁₋₆ alkylthio, (xi) C₁₋₆ alkanoyl, (xii) C₁₋₆ alkanoyloxy, (xiii) amino, (xiv) C₁₋₆ alkylamino, (xv) di-C₁₋₆ alkylamino, (xvi) C₁₋₆ alkanoylamino, (xvii) C₃₋₈ cycloalkyl, (xviii) C₆₋₁₀ aryl and (xix) 5- or 6-membered heterocyclic group containing 1 to 3 nitrogen, sulfur and/or oxygen atom(s) in addition to carbon atoms and further in the case of the (4) and (5) group (xx) a C₁₋₆ alkyl group which may be substituted by one or two of a hydroxy, halogen, amino and carboxyl.

9. A process according to claim 1, wherein R² represents a C₁₋₄ alkoxy-C₁₋₄ alkyl, carbamoyloxy-C₁₋₄ alkyl, carbamoyl-C₆₋₁₀ aryl, thienylthio, S-oxide-thienylthio, furyl or pyridyl group.

10. A process according to claim 1, wherein the compound (II) A is produced by heating a compound of the formula: 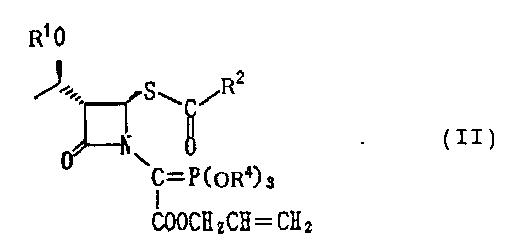 wherein R⁴ represents an alkyl group or an aryl group and the other symbols are as defined in claim 1 in an alcoholic solvent and if necessary removing the protecting group.

11. A process for producing a compound of the formula: 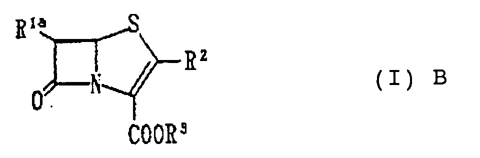 wherein R^{1a} represents a hydroxyalkyl group which may be protected, R² represents an organic group and R³ represents a carboxy-protecting group, which comprises heating a compound of the formula: 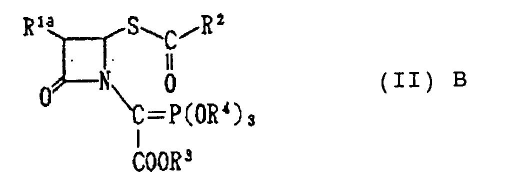 wherein R⁴ represents an alkyl group or an aryl group and the other symbols are as defined above in an alcoholic solvent.

12. A process according to claim 10 or 11, wherein the alcoholic solvent is a primary alcohol of not more than 8 carbon atoms.

13. A process according to claim 10 or 11, wherein the heating is carried out at 50°C to 90°C.

14. A process according to claim 10 or 11, wherein R⁴ represents a C₁₋₆ alkyl group.

15. A process according to claim 10, wherein R¹ represents a hydrogen atom.

16. A process according to claim 10, wherein the compound (II) is produced by reacting a compound of the formula: 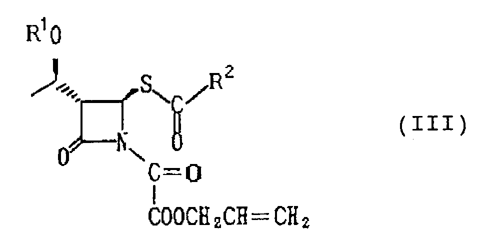 wherein all the symbols are as defined in claim 1 with a compound of the formula:
P(OR⁴)₃
wherein R⁴ is as defined in claim 10.

17. A process according to claim 16, wherein the compound (III) wherein R¹ represents a hydrogen atom is produced by permitting an acid to act upon a compound of the formula: 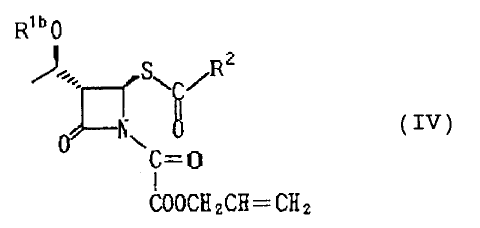 wherein R^{1b} represents a silyl group and R² is as defined in claim 1.

18. A process for producing a compound of the formula: 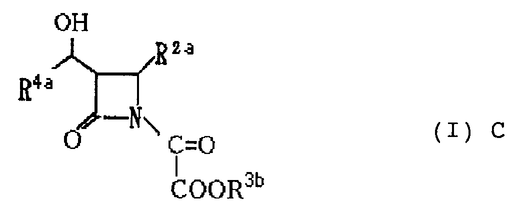 wherein R^{4a} represents an alkyl group, R^{3b} represents a hydrogen atom or a carboxy-protecting group and R^{2a} is as defined in claim 1 or a salt thereof, which comprises permitting an acid to act upon a compound of the formula: 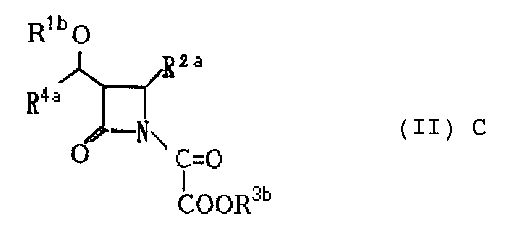 wherein R^{1b} represents a silyl group and the other symbols are as defined above.

19. A process according to claim 17 or 18, wherein an organic solvent is used.

20. A process according to claim 19, wherein the organic solvent is acetonitrile.

21. A process according to claim 17 or 18, wherein the acid is hydrochloric acid.

22. A process according to claim 17 or 18, wherein the silyl group is t-butyldimethylsilyl.

23. A compound of the formula: 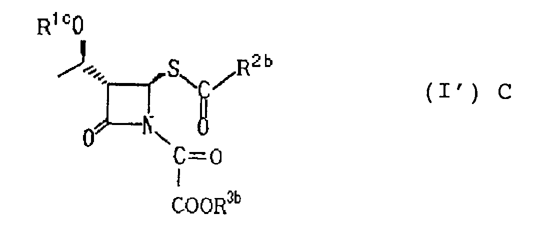 wherein R^{1c} represents a hydrogen atom or a silyl group, CO-R^{2b} represents an acyl group and R^{3b} represents a hydrogen atom or a carboxy-protecting group or a salt thereof.

24. A compound as claimed in claim 23, wherein R^{2b} represents a pyridin-3-yl group and R^{3b} represents an allyl group.

25. A process according to claim 18, wherein R^{2a} is - S-CO-W-R²' wherein W and R²' are as defined in claim 8.

26. A compound as claimed in claim 23, wherein R^{2b} is -W-R²' wherein W and R²' are as defined in claim 8.
